# EUROPEAN PATENT SPECIFICATION

(11) **EP 2 286 832 B1**
(45) Date of publication and mention of the grant of the patent: **23.07.2014**
(21) Application number: 10075497.7
(22) Date of filing: 30.05.2005
(51) Int. Cl.: A61K 39/02, A61P 35/00, A61K 35/74, A61P 37/04

(54) **Bacterial compositions for the treatment of cancer**
Bakterielle Zusammensetzungen zur Behandlung von Krebs
Compositions bactériennes pour le traitement du cancer

(30) Priority: 07.06.2004 US 577206 P
(43) Date of publication of application: 23.02.2011
(62) Divisional of application: 05748702.7
(73) Proprietor: Qu Biologics Inc, Vancouver, BC V5T 4T5 (CA)
(72) Inventor: Gunn Harold D., Vancouver, BC V6H 3P1 (CA)
(74) Representative: Potter Clarkson LLP

(56) References cited:
- EP-A- 1 415 655
- WO-A-95/26742
- WO-A-03/049751
- WO-A1-03/063593
- US-A- 3 928 565
- US-A- 4 329 452
- HROUDA D ET AL: "Immunotherapy of advanced prostate cancer: a phase I/II trial using Mycobacterium vaccae (SRL172).", BRITISH JOURNAL OF UROLOGY OCT 1998, vol. 82, no. 4, October 1998 (1998-10), pages 568-573, XP009045425, ISSN: 0007-1331
- CANN S A HOPTION ET AL: "Dr William Coley and tumour regression: A place in history or in the future.", POSTGRADUATE MEDICAL JOURNAL, vol. 79, no. 938, December 2003 (2003-12), pages 672-680, ISSN: 0032-5473
- CANN S A HOPTION ET AL: "Spontaneous regression: A hidden treasure buried in time", MEDICAL HYPOTHESES, vol. 58, no. 2, February 2002 (2002-02), pages 115-119, ISSN: 0306-9877
- TANG Z Y ET AL: "PRELIMINARY RESULT OF MIXED BACTERIAL VACCINE AS ADJUVANT TREATMENT OF HEPATOCELLULAR CARCINOMA", MEDICAL ONCOLOGY AND TUMOR PHARMACOTHERAPY, STOCKTON PRESS, HANTS, GB, vol. 8, no. 1, 1 March 1991 (1991-03-01), pages 23-28, XP008149065, ISSN: 0736-0118, DOI: 10.1007/BF02988567 [retrieved on 2008-11-16]
- 'Case law of the Boards of Appeal of the EPO', vol. 6TH ED., 2010 * page 351 *
- Medscape reference for Serratia
- Declaration of Dr. Simon Sutcliffe M.D.

## Description

### FIELD OF THE INVENTION

The invention provides in general methods of treating cancers. More specifically, the invention provides methods of treating cancers using bacterial compositions.

### BACKGROUND OF THE INVENTION

More than one in three people in the developed nations are diagnosed with cancer. More than one in four die from it. Therapies for cancer have primarily relied upon treatments such as surgery, chemotherapy, and radiation. These approaches however, while beneficial for some types and stages of cancer, have proved to be of limited efficacy in many common types and stages of cancers. For example, surgical treatment of a tumor requires complete removal of cancerous tissue to prevent reoccurrence. Similarly, radiation therapy requires complete destruction of cancerous cells. This is difficult since, in theory, a single malignant cell can proliferate sufficiently to cause reoccurrence of the cancer. Also, both surgical treatment and radiation therapy are directed to localised areas of cancer, and are relatively ineffective when the cancer metastasises. Often surgery or radiation or both are used in combination with systemic approaches such as chemotherapy. Chemotherapy however has the problem of non-selectivity with the concomitant problem of deleterious side effects, as well as the possibility of the cancer cells developing resistance to the drugs.

Alternative approaches for the treatment of cancers have included therapies that involve stimulation of the immune system such as cytokine therapy (e.g., recombinant interleukin 2 and gamma interferon for kidney cancers), dendritic cell therapy, autologous tumor vaccine therapy, genetically-altered vaccine therapy, lymphocyte therapy, and bacterial vaccine therapy. The above therapies have all proved to be of limited use: cytokine therapy have been effective only in a minority of cases, as has dendritic cell therapy, and the latter is also time consuming and expensive. The diverse vaccine therapies have exhibited highly variable efficacy, and in the case of bacterial vaccines, have proved ineffective in a variety of cancers.

US 3,928,565 teaches the provision of anti-tumor and vaccine preparations comprising cell wall protein component of *Pseudomonas aeruginosa* as active ingredient and a pharmaceutically acceptable carrier, and exemplifies this by use of the composition to treat intraperitoneally transplanted tumor cells in mice.

CANN S A HOPTION ET AL: "Dr William Coley and tumour regression: A place in history or in the future.", POSTGRADUATE MEDICAL JOURNAL, vol. 79, no. 938, December 2003 (2003-12), pages 672-680, and CANN S A HOPTION ET AL: "Spontaneous regression: A hidden treasure buried in time", MEDICAL HYPOTHESES, vol. 58, no. 2, February 2002 (2002-02), pages 115-119, refer how Coley's toxin (a vaccine containing two killed bacteria, Streptococcus pyogenes and Serratia marcescens) was applied to sarcoma patients, as well to treat carcinomas, lymphomas, melanomas and myelomas, breast, ovaries and kidney cancers.

### SUMMARY OF THE INVENTION

The invention provides in part methods of treating cancers in a specific organ or tissue in a subject by administering an antigen of one or more pathogenic bacterial species that are pathogenic in the specific organ or tissue.

In a first aspect, the invention provides a preparation of an antigen of one or more pathogenic bacterial species for use in treating a cancer in a specific organ, tissue or cell in a subject, wherein the preparation elicits an immune response in the subject, and wherein the pathogenic bacterial species is pathogenic in the corresponding specific organ, tissue or cell in a healthy subject, and wherein the antigen of one or more pathogenic bacterial species is provided as one or more attenuated bacteria, and wherein:
the cancer is situated in the kidney and the one or more pathogenic bacterial species are selected from *Escherichia coli, Proteus mirabilis, Proteus vulgatus, Providentia* species, *Morganella* species and *Enterococcus faecalis*; or
the cancer is situated in the lung and the one or more pathogenic bacterial species are selected from *Streptococcus pneumoniae, Moraxella catarrhalis, Mycoplasma pneumoniae, Klebsiella pneumoniae, Haemophilus influenza, Staphylococcus aureus, Chlamydia pneumoniae and Legionella pneumophila;* or
the cancer is situated in the bone and the pathogenic bacterial species is *Staphylococcus aureus;* or
the cancer is situated in the colon and the one or more pathogenic bacterial species are selected from *Bacteroides fragilis, Bacteroides vulgatus, Bacteroides thetaiofaomicron, Clostridium perfringens, Clostridium difficile, Escherichia coli, Salmonella enteritidis, Yersinia enterocolitica* and *Shigella flexneri;* or
the cancer is situated in the prostate and the one or more pathogenic bacterial species are selected from *Escherichia coli, Corynebacterium* species, *Enterococcus faecalis* and *Neisseria gonorrhoeae;* or
the cancer is situated in the skin and the one or more pathogenic bacterial species are selected from *Staphylococcus aureus, Corynebacterium diphtheriae, Corynebacterium ulcerans* and *Pseudomonas aeruginosa;* or
the cancer is situated in the mouth and the one or more pathogenic bacterial species are selected from *Prevotella melaninogenicus,* anaerobic streptococci, viridans streptococci and *Actinomyces* species; or
the cancer is situated in the testicle and the one or more pathogenic bacterial species are selected from *Escherichia coli, Salmonella enteritidis* and *Staphyloccocus aureus*; or
the cancer is situated in the uterus and the one or more pathogenic bacterial species are selected from *Bacteroides fragilis, Escherichia coli, Neisseria gonorrhoeae* and *Chlamydia trachomatis;* or
the cancer is situated in the ovary and the one or more pathogenic bacterial species are selected from *Bacteroides fragilis, Escherichia coli, Neisseria gonorrhoeae* and *Chlamydia trachomatis;* or
the cancer is situated in the vagina and the one or more pathogenic bacterial species are selected from *Bacteroides fragilis* and *Escherichia coli;* or
the cancer is situated in the breast and the pathogenic bacterial species is *Staphyloccocus aureus;* or
the cancer is situated in the gallbladder and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* and *Shigella flexneri;* or
the cancer is situated in the bladder and the pathogenic bacterial species is *Escherichia coli;* or
the cancer is lymphoma associated with the head or neck and the one or more pathogenic bacterial species are selected from *Corynebacterium diptheriae, Corynebacterium ulcerans, Arcanobacterium haemolyticum, Staphylococcus aureus, Pseudomonas aeruginosa, Prevotella melaninogenicus,* anaerobic streptococci, viridans streptococci and *Actinomyces* species; or
the cancer is lymphoma associated with the chest and the one or more pathogenic bacterial species are selected from *Streptococcus pneumoniae, Moraxella catarrhalis, Mycoplasma pneumoniae, Klebsiella pneumoniae, Haemophilus influenzae, Staphylococcus aureus, Chlamydia pneumoniae* and *Legionella pneumophila;* or
the cancer is lymphoma associated with the abdominal cavity and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica, Shigella flexneri, Proteus mirabilis, Proteus vulgatus, Providentia* species, *Morganella* species and *Enterococcus faecalis;* or
the cancer is lymphoma associated with the axilliary or inguinal area and the one ore more pathogenic bacterial species are selected from *Staphylococcus aureus, Corynebacterium diphtheriae, Corynebacterium ulcerans* and *Pseudomonas aeruginosa.*

In another aspect, the invention provides a use of a preparation of an antigen of one or more pathogenic bacterial species in the manufacture of a medicament for treating a cancer in a specific organ, tissue or cell in a subject, wherein the pathogenic bacterial species is pathogenic in the corresponding specific organ, tissue or cell in a healthy subject, and wherein the one or more pathogenic bacterial species and the cancer are as defined in relation to the first aspect of the invention.

If the cancer is lung cancer, the pathogenic bacterial species may be *Streptococcus pneumoniae, Moraxella catarrhalis, Mycoplasma pneumoniae, Klebsiella pneumoniae, Haemophilus influenzae, Staphylococcus aureus, Chlamydia pneumoniae,* or *Legionella pneumophila.*

If the cancer is colon cancer, the pathogenic bacterial species may be *Bacteroides fragilis, Bacteroides vulgatus, Bacteroides thetaiotaomicron, Clostridium perfringens, Clostridium difficile, Escherichia coli, Salmonella enteriditis, Yersinia enterocolitica,* or *Shigella flexneri.*

If the cancer is kidney cancer, the pathogenic bacterial species may be *Escherichia coli, Proteus mirabilis, Proteus vulgatus, Providentia* species, *Morganella* species, or *Enterococcus faecalis.*

If the cancer is prostate cancer, the pathogenic bacterial species may be *Escherichia coli, Corynebacterium* species, *Enterococcus faecalis,* or *Neisseria gonorrhoeae.*

If the cancer is skin cancer, the pathogenic bacterial species may be *Staphylococcus aureus, Streptococcus pyogenes, Corynebacterium diphtheriae, Corynebacterium ulcerans,* or *Pseudomonas aeruginosa.*

If the cancer is bone cancer, the pathogenic bacterial species may be *Staphylococcus aureus.*

If the cancer is mouth cancer, the pathogenic bacterial species may be *Prevotella melaninogenicus,* anaerobic streptococci, viridans streptococci (e.g., *Streptococcus salivarius, Streptococcus sanguis,* or *Streptococcus mutans*), or *Actinomyces* species.

If the cancer is testicle cancer, the pathogenic bacterial species may be *Escherichia coli, Salmonella enteriditis,* or *Staphyloccocus aureus.*

If the cancer is uterine cancer, the pathogenic bacterial species may be *Bacteroides fragilis, Escherichia coli, Neisseria gonorrhoeae,* or *Chlamydia trachomatis.*

If the cancer is ovarian cancer, the pathogenic bacterial species may be *Bacteroides fragilis, Escherichia coli, Neisseria gonorrhoeae,* or *Chlamydia trachomatis.*

If the cancer is vaginal cancer, the pathogenic bacterial species may be *Bacteroides fragilis* or *Escherichia coli.*

If the cancer is breast cancer, the pathogenic bacterial species may be *Staphyloccocus aureus* or *Streptococcus pyogenes.*

If the cancer is gallbladder cancer, the pathogenic bacterial species may be *Bacteroides fragilis, Bacteroides vulgatus, Bacteroides thetaiotaomicron, Clostridium perfringens, Clostridium difficile, Escherichia coli, Salmonella enteriditis, Yersinia enterocolitica,* or *Shigella flexneri.*

If the cancer is bladder cancer, the pathogenic bacterial species may be *Escherichia coli.*

If the cancer is a lymphoma associated with the head and neck, the pathogenic bacterial species may be *Streptococcus Pyogenes, Corynebacterium diptheriae, Corynebacterium ulcerans, Arcanobacterium haemolyticum, Staphylococcus aureus, Pseudomonas aeruginosa, Prevotella melaninogenicus,* anaerobic streptococci, viridans streptococci, or *Actinomyces* species.

If the cancer is a lymphoma associated with the chest, the pathogenic bacterial species may be *Streptococcus pneumoniae, Moraxella catarrhalis, Mycoplasma pneumoniae, Klebsiella pneumoniae, Haemophilus influenzae, Staphylococcus aureus, Chlamydia pneumoniae,* or *Legionella pneumophila.*

If the cancer is a lymphoma associated with the abdominal cavity, the pathogenic bacterial species may be *Bacteroides fragilis, Bacteroides vulgatus, Bacteroides thetaiotaomicron, Clostridium perfringens, Clostridium difficile, Escherichia coli, Salmonella enteriditis, Yersinia enterocolitica, Shigella flexneri, Proteus mirabilis, Proteus vulgatus, Providentia* species, *Morganella* species, or *Enterococcus faecalis.*

If the cancer is a lymphoma associated with the axillary and inguinal area, the pathogenic bacterial species may be *Staphylococcus aureus, Streptococcus pyogenes, Corynebacterium diphtheriae, Corynebacterium ulcerans,* or *Pseudomonas aeruginosa.* In alternative embodiments, the pathogenic bacterial species may be capable of causing infection when present in the specific organ, tissue or cell in a healthy subject, or may have caused an infection in the specific organ, tissue or cell in a healthy subject. In alternative embodiments, the antigen is for administration by administering a whole bacterial species. In alternative embodiments, the subject may be administered at least two or more bacterial species, or at least three or more bacterial species. In alternative embodiments, the subject may further be administered a supplement or an adjuvant. In alternative embodiments, the administering may elicit an immune response in said subject.

A "cancer" or "neoplasm," as used herein, is any unwanted growth of cells serving no physiological function. In general, a cancer cell has been released from its normal cell division control, i.e., a cell whose growth is not regulated by the ordinary biochemical and physical influences in the cellular environment. Thus, "cancer" is a general term for diseases characterized by abnormal uncontrolled cell growth. In most cases, a cancer cell proliferates to form clonal cells that are either benign or malignant. The resulting lump or cell mass, "neoplasm" or "tumor," is generally capable of invading and destroying surrounding normal tissues. By "malignancy" is meant an abnormal growth of any cell type or tissue, that has a deleterious effect in the organism having the abnormal growth. The term "malignancy" or "cancer" includes cell growths that are technically benign but which carry the risk of becoming malignant. Cancer cells may spread from their original site to other parts of the body through the lymphatic system or blood stream in a process known as "metastasis." Many cancers are refractory to treatment and prove fatal. Examples of cancers or neoplasms include, without limitation, transformed and immortalized cells, tumors, carcinomas, in various organs and tissues as described herein or known to those of skill in the art.

A "cell" is the basic structural and functional unit of a living organism. In higher organisms, e.g., animals, cells having similar structure and function generally aggregate into "tissues" that perform particular functions. Thus, a tissue includes a collection of similar cells and surrounding intercellular substances, e.g., epithelial tissue, connective tissue, muscle, nerve. An "organ" is a fully differentiated structural and functional unit in a higher organism that may be composed of different types tissues and is specialized for some particular function, e.g., kidney, heart, brain, liver, etc. Accordingly, by "specific organ, tissue, or cell" is meant herein to include any particular organ, and to include the cells and tissues found in that organ.

An "infection" is the state or condition in which the body or a part of it is invaded by a pathogenic agent (e.g., a microbe, such as a bacterium) which, under favorable conditions, multiplies and produces effects that are injurious (Taber's Cyclopedic Medical Dictionary, 14th Ed., C.L. Thomas, Ed., F.A. Davis Company, PA, USA). An infection may not always be apparent clinically, or may result in localized cellular injury. Infections may remain localized, subclinical, and temporary if the body's defensive mechanisms are effective. A local infection may persist and spread to become an acute, a subacute, or a chronic clinical infection or disease state. A local infection may also become systemic when the pathogenic agent gains access to the lymphatic or vascular system (On-Line Medical Dictionary, http://cancerweb.ncl.ac.uk/omd/. Localized infection is usually accompanied by inflammation, but inflammation may occur without infection.

"Inflammation" is the characteristic tissue reaction to injury (marked by swelling, redness, heat, and pain), and includes the successive changes that occur in living tissue when it is injured. Infection and inflammation are different conditions, although one may arise from the other (Taber's Cyclopedic Medical Dictionary, *supra*). Accordingly, inflammation may occur without infection and infection may occur without inflammation (although inflammation typically results from infection by pathogenic bacteria).

A "subject" is an animal, e.g, a mammal, to whom the specific pathogenic bacteria, bacterial antigens, or compositions thereof of the invention may be administered. Accordingly, a subject may be a patient, e.g., a human, suffering from a cancer, or suspected of having a cancer, or at risk for developing a cancer. A subject may also be an experimental animal, e.g., an animal model of a cancer. In some embodiments, the terms "subject" and "patient" may be used interchangeably, and may include a human, a non-human mammal, a non-human primate, a rat, mouse, dog, etc. A healthy subject may be a human who is not suffering from a cancer or suspected of having a cancer, or who is not suffering from a chronic disorder or condition. A "healthy subject" may also be a subject who is not immunocompromised. By immunocompromised is meant any condition in which the immune system functions in an abnormal or incomplete manner, for example, a condition which prevents or reduces a full and normal immune response, or renders the immunocompromised subject more susceptible to microbial (e.g., bacterial) infection. Immunocompromisation may be due to disease, certain medications, or conditions present at birth. Immunocompromised subjects may be found more frequently among infants, children, the elderly, individuals undergoing extensive drug or radiation therapy.

An "immune response" includes, but is not limited to, one or more of the following responses in a mammal: induction of antibodies, B cells, T cells (including helper T cells, suppressor T cells, cytotoxic T cells, γδ T cells) directed specifically to the antigen(s) in a composition or vaccine, following administration of the composition or vaccine. An immune response to a composition or vaccine thus generally includes the development in the host animal of a cellular and/or antibody-mediated response to the composition or vaccine of interest. In general, the immune response will result in slowing or stopping the progression of cancer in the animal.

### BRIEF DESCRIPTION OF THE DRAWINGS

**Figure 1** shows a survival curve for patients initially diagnosed with stage 3B or 4 inoperable lung cancer (all patients).
**Figure 2** shows a survival curve for patients initially diagnosed with stage 3B or 4 inoperable lung cancer (patients treated for over 2 months with MRV).

### DETAILED DESCRIPTION OF THE INVENTION

The invention provides in part compositions including whole bacterial species, as well as components thereof, for the treatment of cancer and methods for using the same. The inventor has surprisingly found that administration of pathogenic bacterial species that are pathogenic in a particular cell, tissue, or organ is effective in treating cancer of that specific cell, tissue, or organ.

Based on observations from treating patients, it was found that administering compositions including many of the most common pathogenic bacteria that cause lung and upper respiratory tract infection and/or including *Staphylococcus aureus,* one of the most common causes of skin infection, was surprisingly and unexpectedly effective in improving the clinical course of lung cancer, lymphoma (cancer of the lymph glands) and malignant melanoma (a type of skin cancer). Similarly, it was surprisingly and unexpectedly found that administering a composition including pathogenic *Escherichia coli,* which is a common cause of colon, kidney, bladder, prostate, uterine and ovarian infection was effective in improving the clinical course of colon, ovarian, kidney and prostate cancer.

These results indicate that a bacterial composition including antigens of pathogenic bacterial species that most commonly cause infection in a particular cell, tissue, or organ will be the most effective formulation for treating a particular cancer. For example, lung cancer is most effectively treated with a bacterial composition including pathogenic bacterial species that commonly cause lower respiratory tract infection, while kidney cancer is most effectively treated with a bacterial composition including pathogenic bacterial species that commonly cause kidney infections.

In some embodiments, the pathogenic bacterial compositions may be used for treating primary cancer sites and/or sites of metastasis. Thus, for example, the pathogenic bacterial compositions may be used for treatment of both the primary cancer and the mestastatic site. The composition may be directed to the treatment of each of these sites, or may be a combined composition for both the primary cancer and the metastatic site(s). For example, to treat kidney cancer that has metastasized to the lung and bone, three different compositions including pathogenic bacterial species that are pathogenic in kidney, lung and bone, or a combined composition thereof may be used. In embodiments, the compositions may be administered in different locations at the same time or at different times.

For example, for lung cancer with metastasis to the bone, in alternative embodiments, both a pathogenic bacterial compositions including bacteria which commonly cause lung infection and a pathogenic bacterial composition including bacteria which commonly cause bone infection may be used. Similarly, for colon cancer with metastasis to the lungs, both a pathogenic bacterial composition including bacteria which commonly cause colon infection and a pathogenic bacterial composition including bacteria which commonly cause lung infection may be used; for prostate cancer with metastasis to the bones, both a pathogenic bacterial composition including bacteria which commonly cause prostate infection and a pathogenic bacterial composition including bacteria that commonly cause bone infection may be used.

The following list provides some non-limiting examples of primary cancers and their common sites for secondary spread (metastases):

| Primary cancer | Common sites for metastases |
|---|---|
| prostate | bone |
| breast | bone, lungs, skin |
| lung | bone, |
| colon | liver, lungs, bone |
| kidney | lungs, bone |
| pancreas | liver, lungs, bone |
| melanoma | lungs |
| uterus | lungs, bones, ovaries |
| ovary | liver, lung |
| bladder | bone, lung |

In some embodiments, the pathogenic bacterial compositions may be used for treating or preventing cancers at primary sites or preventing metastasis. For example, in long-term smokers, a lung cancer vaccine (including bacteria which commonly cause lung infection) may be used to appropriately stimulate the immune system to defend against the development of cancer within the lung tissue. As another example, a breast cancer vaccine (including bacteria which commonly cause breast infection) could be used to prevent breast cancer in women with a strong family history of breast cancer or a genetic predisposition. In alternative embodiments, a vaccine including bacteria which commonly cause bone infection may be used to prevent bone metastases in a patient with prostate cancer. In further alternative embodiments, a vaccine including bacteria which commonly cause lung infection may be used to prevent lung metastases in a patient with malignant melanoma.

Various alternative embodiments and examples of the invention are described herein. These embodiments and examples are illustrative and should not be construed as limiting the scope of the invention.

### Cancers

Most cancers fall within three broad histological classifications: carcinomas, which are the predominant cancers and are cancers of epithelial cells or cells covering the external or internal surfaces of organs, glands, or other body structures (e.g., skin, uterus, lung, breast, prostate, stomach, bowel), and which tend to mestastasize; sarcomas, which are derived from connective or supportive tissue (e.g., bone, cartilage, tendons, ligaments, fat, muscle); and hematologic tumors, which are derived from bone marrow and lymphatic tissue. Carcinomas may be adenocarcinomas (which generally develop in organs or glands capable of secretion, such as breast, lung, colon, prostate or bladder) or may be squamous cell carcinomas (which originate in the squamous epithelium and generally develop in most areas of the body). Sarcomas may be osteosarcomas or osteogenic sarcomas (bone), chondrosarcomas (cartilage), leiomyosarcomas (smooth muscle), rhabdomyosarcomas (skeletal muscle), mesothelial sarcomas or mesotheliomas (membranous lining of body cavities), fibrosarcomas (fibrous tissue), angiosarcomas or hemangioendotheliomas (blood vessels), liposarcomas (adipose tissue), gliomas or astrocytomas (neurogenic connective tissue found in the brain), myxosarcomas (primitive embryonic connective tissue), or mesenchymous or mixed mesodermal tumors (mixed connective tissue types). Hematologic tumors may be myelomas, which originate in the plasma cells of bone marrow; leukemias which may be "liquid cancers" and are cancers of the bone marrow and may be myelogenous or granulocytic leukemia (myeloid and granulocytic white blood cells), lymphatic, lymphocytic, or lymphoblastic leukemias (lymphoid and lymphocytic blood cells) or polycythemia vera or erythremia (various blood cell products, but with red cells predominating); or lymphomas, which may be solid tumors and which develop in the glands or nodes of the lymphatic system, and which may be Hodgkin or Non-Hodgkin lymphomas. In addition, mixed type cancers, such as adenosquamous carcinomas, mixed mesodermal tumors, carcinosarcomas, or teratocarcinomas also exist.

Cancers may also be named based on the organ in which they originate i.e., the "primary site," for example, cancer of the breast, brain, lung, liver, skin, prostate, testicle, bladder, colon and rectum, cervix, uterus, etc. This naming persists even if the cancer metastasizes to another part of the body, that is different from the primary site.

Cancers named based on primary site may be correlated with histological classifications. For example, lung cancers are generally small cell lung cancers or non-small cell lung cancers, which may be squamous cell carcinoma, adenocarcinoma, or large cell carcinoma; skin cancers are generally basal cell cancers, squamous cell cancers, or melanomas. Lymphomas may arise in the lymph nodes associated with the head, neck and chest, as well as in the abdominal lymph nodes or in the axillary or inguinal lymph nodes. Identification and classification of types and stages of cancers may be performed by using for example information provided by the Surveillance, Epidemiology, and End Results (SEER) Program of the National Cancer Institute
(http://seer.cancer.gov/publicdata/access.html), which is an authoritative source of information on cancer incidence and survival in the United States and is recognized around the world. The SEER Program currently collects and publishes cancer incidence and survival data from 14 population-based cancer registries and three supplemental registries covering approximately 26 percent of the US population. The program routinely collects data on patient demographics, primary tumor site, morphology, stage at diagnosis, first course of treatment, and follow-up for vital status, and is the only comprehensive source of population-based information in the United States that includes stage of cancer at the time of diagnosis and survival rates within each stage. Information on more than 3 million in situ and invasive cancer cases is included in the SEER database, and approximately 170,000 new cases are added each year within the SEER coverage areas. The incidence and survival data of the SEER Program may be used to access standard survival for a particular cancer site and stage. For example, to ensure an optimal comparison group, specific criteria may be selected from the database, including date of diagnosis and exact stage (in the case of the lung cancer example herein, the years were selected to match the time-frame of the retrospective review, and stage 3B and 4 lung cancer were selected).

### Bacteria and Bacterial Colonizations and Infections

Most animals are colonized to some degree by extrinsic organisms, such as bacteria, which generally exist in symbiotic or commensal relationships with the host animal. Thus, many species of generally harmless bacteria are normally found in healthy animals, and are usually localized to specific organs and tissues. Often, these bacteria aid in the normal functioning of the body. For example, in humans, symbiotic *Lactobacilli acidophilus* may be found in the intestine, where they assist in food digestion.

Infectious bacteria (pathogenic bacteria) have in general a parasitic relationship with the host animal and cause disease in healthy subjects, with the results ranging from mild to severe infection to death. However, whether or not a bacterium is pathogenic (i.e., causes infection) depends to some extent on factors such as the route of entry and access to specific host cells, tissues, or organs; the intrinsic virulence of the bacterium; the amount of the bacteria present at the site of potential infection; or the immune status of the host animal (e.g., healthy or immunocompromised). Thus, bacteria that are normally harmless can become pathogenic given favorable conditions for infection, and even the most virulent bacterium requires specific circumstances to cause infection. For example, bacterial species present on skin remain harmless on the skin, but when present in a normally sterile space, such as in the capsule of a joint, or the peritoneum, will likely cause infection. It will be understood by one of skill in the medical arts that a normally harmless bacterium (e.g., a symbiotic or commensal bacterium) may be capable of causing infection if present in a cell, tissue, or organ in which it is not normally found; in some embodiments, such bacteria are not considered bacteria that "cause infection" or are "pathogenic" and as such are specifically excluded from the methods and compositions of the invention even if under restricted, abnormal circumstances, they are capable of causing infection. In some embodiments, bacteria that do not normally cause infection in a healthy subject are specifically excluded from the methods and compositions of the invention.

Some bacteria that are normally present in or on a host animal do not cause infection, but are capable of causing inflammation. For example, *Propionibacterium acnes* (*P. acnes;* formerly known as *Corynebacterium parvum* or *C. parvum*)*,* which normally lives on the skin, can grow in plugged hair follicles and cause acne. Acne is not considered an infectious disease, and it is believed that enzymes and inflammatory mediators produced by *P. acnes* diffuse into the surrounding skin, causing inflammation.

Pathogenic bacteria generally cause infections in specific cells, tissues, or organs (e.g., localized infections) in otherwise healthy subjects. Examples of pathogenic bacteria that commonly cause infections in various parts of the body are listed below; it will be understood that these examples are not intended to be limiting and that a skilled person would be able to readily recognize and identify infectious or pathogenic bacteria that cause infections in various organs and tissues in healthy adults (and recognize the relative frequency of infection with each bacterial species) based on the knowledge in the field as represented, for example, by the following publications: Manual of Clinical Microbiology 8th Edition, Patrick Murray, Ed., 2003, ASM Press American Society for Microbiology, Washington DC, USA: Chapter 42, Escherichia, Shigella, and Salmonella, C.A. Bopp, F.R. Brenner, P.J. Fields, J.G. Wells, N.A. Strockbine, pp: 654-670; Mandell, Douglas, and Bennett's Principles and Practice of Infectious Diseases 5th Edition, G. L. Mandell, J.E. Bennett, R. Dolin, Eds., 2000, Churchill Livingstone, Philadelphia, PA, USA: Chapter 71, Acute Meningitis, A.R. Turkel, Pp: 959-995; Chapter 62, Urinary Tract Infections J.D. Sobel, D. Kaye Pp: 773-805; Chapter 57, Acute Pneumonia G.R. Donowitz, G.L. Mandell Pp: 717-742; Chapter 81 Principles and Syndromes of Enteric Infection R.L. Guerrant, T.S. Steiner Pp:1076-1093, all of which are incorporated by reference herein.

Lung infections (e.g., pneumonia) are generally caused by *Streptococcus pneumoniae, Moraxella catarrhalis* (previously referred to as *Neisseria catarrhalis*), or *Mycoplasma pneumoniae,* which generally account for over 90% of the cases of pneumonia. Lung infections may also be caused by *Klebsiella pneumoniae, Haemophilus influenzae, Staphylococcus aureus, Chlamydia pneumoniae,* or *Legionella pneumophila.*

Bowel infections are generally the consequence of breakdown of normal protective mechanisms and result in mixed infections that usually include mixed anaerobic gram negative bacilli, for example *Bacteroides fragilis, Bacteroides vulgatus,* or *Bacteroides thetaiotaomicron.* Other anaerobes involved in bowel infection include *Clostridium perfringens* and *Clostridium difficile,* neither of which is normally resident in bowel flora. The most common species of Clostridium found in human bowel flora are generally *Clostridium innocuum* and *Clostridium ramosum.* Bowel infections usually, but not always, also include enteric bacteria capable of survival in air, for example, *Escherichia coli.* Additional bacteria that may be involved in bowel infections include *Salmonella enteriditis, Yersinia enterocolitica, Shigella flexneri, Enterococcus faecalis* (a gram positive bacteria of low pathogenicity often found in mixed infections) or *Campylobacter jejuni* (a normal flora bacteria in chickens and some dogs, but capable of causing bowel infections in humans).

Generally, bacterial infections of the bladder, kidney, testicle, or prostate are caused by *Escherichia coli.* Other bacteria involved in kidney infections include *Proteus mirabilis, Proteus vulgatus, Providentia* species, *Morganella* species, or *Enterococcus faecalis.* Testicle infections may also be caused by *Salmonella enteriditis* or *Staphylococcus aureus.* Prostate infections may also result from bacteria associated with normal urethral flora including *Corynebacterium* species or *Enterococcus faecalis.* Prostate infection may also result from direct invasion by *Neisseria gonorrhoeae.*

Generally, skin infections involve *Staphylococcus aureus* or *Streptococcus pyogenes.* Skin infections may also result from *Corynebacterium diphtheriae, Corynebacterium ulcerans,* or *Pseudomonas aeruginosa* (e.g., in burns). Bone infections are generally caused by *Staphylococcus aureus.*

The mouth and colon are the two most heavily colonized parts of the body. Mouth infections generally involve endogenous "normal flora". The major anaerobic bacteria in the mouth are *Prevotella melaninogenicus* and *Anaerobic streptococci.* Mouth infections may also be associated with *Actinomyces* species, or with the viridans Streptococci *Streptococcus sanguis* (frequently associated with endocarditis), *Streptococcus salivarius,* or *Streptococcus mutans* (a major cause of dental caries).

Bacterial infections of the uterus, ovary, and vagina are generally due to *Bacteroides fragilis* and *Escherichia coli.* Uterine or ovarian infections may also be due to *Neisseria gonorrhoeae* or *Chlamydia trachomatis.*

Breast infections are generally caused by *Staphylococcus aureus* or *Streptococcus pyogenes.*

Gallbladder infections are generally due to *Escherichia coli* or oral streptococci (as in mouth infections).

It will be understood by a skilled person that bacterial species are classified operationally as collections of similar strains (which generally refers to groups of presumed common ancestry with identifiable physiological but usually not morphological distinctions, and which may be identified using serological techniques against bacterial surface antigens). Thus, each bacterial species (e.g., *Streptococcus pneumoniae*) has numerous strains (or serotypes), which differ in their ability to cause infection or differ in their ability to cause infection in a particular organ/site. For example, although there are at least 90 serotypes of *Streptococcus pneumoniae,* serotypes 1, 3, 4, 7, 8, and 12 are most frequently responsible for pneumococcal disease in humans. As a second example to illustrate this principle, there are many serotypes of *Escherichia coli,* the most common cause of colon and urinary tract (kidney and bladder) infection. E. coli serotypes which commonly cause colon infection include 06:H16,08:H9,015:H22,025:H-, 028ac:H-, 0136:H-, 0157:H7, 051:H11, and 077:H18. On the other hand, the most common E. coli serotypes to cause urinary tract infection are O groups 1, 2, 4, 6, 7, 18, 25, 50 and 75 (Infectious Diseases: A Treatise of Infectious Diseases; Editors: P. Hoeprich, M.C. Jordan, A. Ronald (1994) J.B. Lippincott Company: Philidelphia p. 602)..

### Bacterial Compositions, Dosages, And Administration

The compositions of the invention include antigens of pathogenic bacterial species that are pathogenic in a specific cell, tissue, or organ. The compositions may include whole bacterial species, or may include extracts or preparations of the pathogenic bacterial species of the invention, such as cell wall or cell membrane extracts or whole cell extracts. The compositions may also include one or more isolated antigens from one or more of the pathogenic bacterial species of the invention; in some embodiments, such compositions may be useful in situations where it may be necessary to precisely administer a specific dose of a particular antigen, or may be useful if administering a whole bacterial species or components thereof (e.g., toxins) may be harmful. Pathogenic bacterial species may be available commercially (from, for example, ATCC (Manassas, VA, USA), or may be clinical isolates from subjects having a bacterial infection of a cell, tissue, or organ (e.g., pneumonia).

The bacterial compositions of the invention can be provided alone or in combination with other compounds (for example, nucleic acid molecules, small molecules, peptides, or peptide analogues), in the presence of a liposome, an adjuvant, or any pharmaceutically acceptable carrier, in a form suitable for administration to mammals, for example, humans. As used herein "pharmaceutically acceptable carrier" or "excipient" includes any and all solvents, dispersion media, coatings, antibacterial and antifungal agents, isotonic and absorption delaying agents, and the like that are physiologically compatible. The carrier can be suitable for any appropriate form of administration, including subcutaneous, intravenous, parenteral, intraperitoneal, intramuscular, sublingual, inhalational, or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound (i.e., the specific bacteria, bacterial antigens, or compositions thereof of the invention), use thereof in the pharmaceutical compositions of the invention is contemplated. Supplementary active compounds can also be incorporated into the compositions.

If desired, treatment with bacterial antigens according to the invention may be combined with more traditional and existing therapies for cancer, such as chemotherapy, radiation therapy, surgery, etc., or with any other therapy intended to stimulate the immune system or otherwise benefit the subject, such as nutrients, vitamins and supplements. For example, antioxidants, Vitamins A, D, E, C, and B complex; Selenium; Zinc; Co-enzyme Q₁₀, Ground Flaxseed; Garlic; Lycopene; Milk Thistle; Melatonin; Cimetidine; Indomethacin; or COX-2 Inhibitors (e.g., Celebrex (celecoxib) or Vioxx (rofecoxib)) may be also be administered to the subject.

Conventional pharmaceutical practice may be employed to provide suitable formulations or compositions to administer the compounds to subjects suffering from a cancer. Any appropriate route of administration may be employed, for example, parenteral, intravenous, subcutaneous, intramuscular, intracranial, intraorbital, ophthalmic, intraventricular, intracapsular, intraspinal, intrathecal, intracisternal, intraperitoneal, intranasal, inhalational, aerosol, topical, or oral administration. Therapeutic formulations may be in the form of liquid solutions or suspensions; for oral administration, formulations may be in the form of tablets or capsules; and for intranasal formulations, in the form of powders, nasal drops, or aerosols.

Methods well known in the art for making formulations are found in, for example, "Remington's Pharmaceutical Sciences" (20th edition), ed. A. Gennaro, 2000, Mack Publishing Company, Easton, PA. Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel. For therapeutic or prophylactic compositions, the pathogenic bacterial species are administered to an individual in an amount effective to stop or slow progression or metastasis of the cancer, or to increase survival of the subject (relative to for example prognoses derived from the SEER database) depending on the disorder.

An "effective amount" of a pathogenic bacterial species or antigen thereof according to the invention includes a therapeutically effective amount or a prophylactically effective amount. A "therapeutically effective amount" refers to an amount effective, at dosages and for periods of time necessary, to achieve the desired therapeutic result, such as reduction or elimination of the cancer cells, tissues, organs, or tumors, or an increase in survival time beyond that which is expected using for example the SEER database. A therapeutically effective amount of a pathogenic bacterial species or antigen thereof may vary according to factors such as the disease state, age, sex, and weight of the individual, and the ability of the compound to elicit a desired response in the individual. Dosage regimens may be adjusted to provide the optimum therapeutic response. A therapeutically effective amount may also be one in which any toxic or detrimental effects of the pathogenic bacterial species or antigen thereof are outweighed by the therapeutically beneficial effects. A "prophylactically effective amount" refers to an amount effective, at dosages and for periods of time necessary, to achieve the desired prophylactic result, such as reduction or elimination of the cancer cells, tissues, organs, or tumors, or an increase in survival time beyond that which is expected using for example the SEER database. Typically, a prophylactic dose is used in subjects prior to or at an earlier stage of cancer, so that a prophylactically effective amount may be less than a therapeutically effective amount. An exemplary range for therapeutically or prophylactically effective amounts of one or more pathogenic bacterial species may be about 1 million to 8000 million organisms per ml, or may be 100 million to 7000 million organisms per ml, or may be 500 million to 6000 million organisms per ml, or may be 1000 million to 5000 million organisms per ml, or may be 2000 million to 4000 million organisms per ml, or any integer within these ranges. The total concentration of bacteria per ml may range from 20 million to 8000 million organisms per ml, or may be 50 million to 7000 million organisms per ml, or may be 100 million to 6000 million organisms per ml, or may be 500 million to 5000 million organisms per ml, or may be 1000 million to 4000 million organisms per ml, or any integer within these ranges. The range for therapeutically or prophylactically effective amounts of antigens of a pathogenic bacterial species may be any integer from 0.1 nM-0.1M, 0.1 nM-0.05M, 0.05 nM-15µM or 0.01 nM-10µM.

It is to be noted that dosage concentrations and ranges may vary with the severity of the condition to be alleviated, or may vary with the subject's immune response. In general, the goal is to achieve an adequate immune response (e.g, a local skin reaction, e.g, from 1 inch to 3 inch; or a systemic fever response immune response (e.g., systemic symptoms of fever and sweats). The dose required to achieve an appropriate immune response varies depending on the individual (and their immune system) and the response desired. For example, if the goal is to achieve a 2 inch local skin reaction, the total bacterial composition dose may range from 20 million bacteria (i.e., 0.01 ml of a vaccine with a concentration of 2,000 million organisms per ml) to more than 800 million bacteria (i.,e., 0.40 ml of a vaccine with a concentration of 2,000 million organisms per ml). The concentrations of individual bacterial species or antigens thereof within a composition may also be considered, since individuals may vary in their response to various bacterial species. For example, if the concentration of one particular pathogenic bacterial species or antigen thereof is much higher relative to the concentrations of other pathogenic bacterial species in the vaccine, then the local skin reaction of an individual may be likely due to its response to this specific bacterial species. In some embodiments, the immune system of an individual may respond more strongly to one bacterial species within a composition than another, so the dosage or composition may be adjusted accordingly for that individual.

For any particular subject, specific dosage regimens may be adjusted over time (e.g, daily, weekly, monthly) according to the individual need and the professional judgement of the person administering or supervising the administration of the compositions. For example, the compositions may be administered every second day or three times a week. In another example, an initial prophylactic dose of 0.05 ml may be administered subcutaneously, following by increases from 0.05-0.1 ml at 4 to 7 day intervals, until a maximum dose of 0.5-1.0 ml. In acute conditions, for example, an initial dose of 0.02 ml may be given followed by increments of 0.02-0.05 ml at 3 to 5 day intervals. Due to the variation in individual sensitivities, doses may be increased at different rates for different individuals. Maintenance doses of 0.5 ml may be given at appropriate (e.g., weekly) intervals.

Dosage ranges set forth herein are exemplary only and do not limit the dosage ranges that may be selected by medical practitioners. The amount of active compound (e.g., pathogenic bacterial species or antigens thereof) in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

In the case of vaccine formulations, an immunogenically effective amount of a compound of the invention can be provided, alone or in combination with other compounds, with an immunological adjuvant. The compound may also be linked with a carrier molecule, such as bovine serum albumin or keyhole limpet hemocyanin to enhance immunogenicity. A "vaccine" is a composition that includes materials that elicit a desired immune response. A vaccine may select, activate or expand memory B and T cells of the immune system to, for example, reduce or eliminate the growth or proliferation of cancerous cells or tissue. In some embodiments, the specific pathogenic bacteria, bacterial antigens, or compositions thereof of the invention are capable of eliciting the desired immune response in the absence of any other agent, and may therefore be considered to be a "vaccine." In some embodiments, a vaccine includes a suitable carrier, such as an adjuvant, which is an agent that acts in a non-specific manner to increase the immune response to a specific antigen, or to a group of antigens, enabling the reduction of the quantity of antigen in any given vaccine dose, or the reduction of the frequency of dosage required to generate the desired immune response. A "bacterial vaccine," as used herein, is a vaccine generally including weakened (attenuated) or dead bacteria capable of inducing an immune response against the disease or infection normally caused by the bacteria in the vaccine. In some embodiments, a bacterial vaccine may include live bacteria that are of less virulent strains, and therefore cause a less severe infection.

A killed bacterial vaccine composition may be made as follows. The bacteria may be grown in suitable media, and washed with physiological salt solution. The bacteria may then be centrifuged, resuspended in salt solution, and killed with phenol. The suspensions may be standardized by direct microscopic count, mixed in required amounts, and stored in appropriate containers, which may be tested for safety, shelf life, and sterility in an approved manner. In addition to the pathogenic bacterial species and/or antigens thereof, a killed bacterial vaccine suitable for administration to humans may include 0.4% phenol preservative, 0.9% sodium chloride. Optionally, the bacterial vaccine may also include trace amounts of brain heart infusion (beef), peptones, yeast extract, agar, sheep blood, dextrose, and/or sodium phosphate. The bacterial vaccine may be used for subcutaneous injection.

In for example bacterial vaccines (e.g., killed bacterial vaccines), the concentrations of specific bacterial species may be about 1 million to 8000 million organisms per ml, or may be 100 million to 7000 million organisms per ml, or may be 500 million to 6000 million organisms per ml, or may be 1000 million to 5000 million organisms per ml, or may be 2000 million to 4000 million organisms per ml, or any integer within these ranges. The total concentration of bacteria per ml may range from 20 million to 8000 million organisms per ml, or may be 50 million to 7000 million organisms per ml, or may be 100 million to 6000 million organisms per ml, or may be 500 million to 5000 million organisms per ml, or may be 1000 million to 4000 million organisms per ml, or any integer within these ranges.

In some embodiments, a optimal killed bacterial vaccine for lung cancer may be:

| | bacteria per ml |
|---|---|
| Streptococcus pneumoniae | 600 million |
| Moraxella catarrhalis | 400 million |
| Mycoplasma pneumoniae | 400 million |
| Klebsiella pneumoniae | 200 million |
| Haemophilus influenzae | 200 million |
| Staphylococcus aureus | 200 million |
| total: | 2,000 million |

In some embodiments, a bacterial vaccine for treating particular cancer (e.g., lung cancer) may include specific strain or serotypes that most commonly cause infection (e.g., pneumonia) in that tissue, cell, or organ.

In general, the pathogenic bacterial species and antigens thereof of the invention should be used without causing substantial toxicity. Toxicity of the compounds of the invention can be determined using standard techniques, for example, by testing in cell cultures or experimental animals and determining the therapeutic index, i.e., the ratio between the LD50 (the dose lethal to 50% of the population) and the LD100 (the dose lethal to 100% of the population). In some circumstances however, such as in severe disease conditions, it may be necessary to administer substantial excesses of the compositions.

### EXAMPLE 1

### Bacterial Compositions

Approximately 750 cancer patients, of all cancer types and stages, have been treated with mixed bacterial vaccines in blinded studies where the patients were not aware of the compositions of the vaccines. Three mixed bacterial vaccines have been used, as follows:
1. The Bayer Corporation MRV™ "Bayer MRV" (Hollister-Steir Laboratories, Spokane, WA, U.S.A.), containing the following bacterial species:

| | Organisms per ml |
|---|---|
| *Staphylococcus aureus* | 1200 million |
| viridans and non-hemolytic Streptococci | 200 million |
| *Streptococcus pneumoniae* | 150 million |
| *Moraxella (Neisseria) catarrhalis* | 150 million |
| *Klebsiella pneumoniae* | 150 million |
| *Haemophilus influenzae* | 150 million |

This vaccine was produced for the following indications: rhinitis, infectious asthma, chronic sinusitis, nasal polyposis and chronic serous otitis media. Cancer treatment was not indicated as an intended use for this vaccine.
2. Stallergenes MRV "Stallergenes MRV" (Laboratories des Stallergenes, S.A., Fresnes, France, containing the following:

| | Organisms per ml |
|---|---|
| *Staphylococcus aureus* | 600 million |
| *Staphylococcus albus* | 600 million |
| non-hemolytic Streptococci | 200 million |
| *Streptococcus pneumoniae* | 150 million |
| *Moraxella (Neisseria) catarrhalis* | 150 million |
| *Klebsiella pneumoniae* | 150 million |
| *Haemophilus influenzae* | 150 million |

This vaccine was produced for the same indications as the MRV vaccine i.e., recurrent respiratory tract infections, and listed cancer as a contraindication.
3. Polyvaccinum Forte (Biomed S.A., Krakow, Poland), containing the following:

| | Organisms per ml |
|---|---|
| *Staphylococcus aureus* | 500 million |
| *Staphylococcus epidermidis* | 500 million |
| *Escherichia coli* | 200 million |
| *Corynebacterium pseudodiphtheriticum* | 200 million |
| *Streptococcus pyogenes* | 100 million |
| *Streptococcus salivarius* (viridans Streptococci) | 100 million |
| *Streptococcus pneumoniae* | 100 million |
| *Moraxella (Neisseria) catarrhalis* | 100 million |
| *Klebsiella pneumoniae* | 100 million |
| *Haemophilus influenzae* | 100 million |

This vaccine was produced for chronic and recurrent inflammatory conditions of the upper and lower respiratory tract, including rhinopharyngitis, recurrent laryngitis, tracheitis, bronchitis, otitis media, chronic and recurrent neuralgia of trigeminal and occipital nerve, ischialgia, brachial plexitis and intercostals neuralgia, as well as chronic cystoureteritis, vaginitis, adnexitis, and endometrium inflammation. Cancer treatment was not indicated as an intended use for this vaccine.

### Administration

The bacterial compositions (vaccines) were a suspension of killed bacterial cells and therefore, the suspensions were gently shaken prior to use to ensure uniform distribution prior to withdrawing dose from vial, and administered subcutaneously three times a week on Mondays, Wednesdays, and Fridays for at least 6 months. The dose of vaccine required was determined by the adequacy of the immune reaction to the vaccine. Beginning with a very small dose (0.05cc), the dose was gradually increased (by 0.01-0.02cc each time) until an adequate immune reaction was achieved. The goal was to achieve a one to two inch diameter round patch of pinkness/redness at the injection site, indicating adequate immune stimulation. Once this reaction was achieved, the dose was maintained at the level required to achieve this reaction. If the reaction was significantly less than two inches (e.g., half an inch) the dose was increased, if it was significantly more than two inches (e.g., three inches), the dose was decreased. This local reaction generally occurs within the first 24 hours after the injection. Patients were asked to check for this reaction and, if present, to measure or mark it. The maintenance dose required to achieve an adequate immune reaction varies considerably, depending on the individual's immune response - as little as 0.01cc for some people, as much as 0.40cc for others. The maximum dose given is 0.40cc. The vaccine must be stored in a refrigerator (2° to 8°C). The usual site for injection is the upper arms, the thighs or the abdomen. The exact site of each injection was varied so that it was not given in sites in which pinkness/redness was still present. There are no known contraindications to the vaccines.

### Lung Cancer

Patients qualified for the lung cancer study if they were initially diagnosed with stage 3B or 4-lung (inoperable) cancer. Lung cancer staging was performed using standard methods as for example described in AJCC: Cancer Staging Handbook (sixth edition) 2002; Springer-Verlag New York: Editors: Fredrick Greene, David Page and Irvin Fleming, or in International Union Against Cancer: TNM Classification of Malignant Tumors (sixth edition) 2002; Wiley-Liss Geneva Switzerland: Editors: L.H. Sobin and C.H. Wittekind. For example, lung cancers may be classified as follows:

### TNM LUNG CLINICAL AND PATHOLOGICAL CLASSIFICATION:

- **T**: **PRIMARY TUMOUR**
- TX: Primary tumour cannot be assessed, ortumour proven by the presence of malignant cells in sputum or bronchial washings but not visualized by imaging or bronchoscopy
- Tis: Carcinoma in situ
- T0: No evidence of primary tumour
- T1: Tumour 3 cm or less in greatest dimension, surrounded by lung or visceral pleura, without bronchoscopic evidence of invasion more proximal than the lobar bronchus (*ie*, not in the main bronchus)
- T2: Tumour with *an*y of the following features of size or extent: More than 3 cm in greatest dimension Involves main bronchus, 2 cm or more distal to the carina Invades visceral pleura Associated with atelectasis or obstructive pneumonitis that extends to the hilar region but does not involve the entire lung
- T3: Tumour of any size that directly invades any of the following: chest wall (including superior sulcus tumours), diaphragm, mediastinal pleura, parietal pericardium; ortumour in the main bronchus less than 2 cm distal to the carina but without involvement of the carina; or associated atelectasis or obstructive pneumonitis of the entire lung
- T4: Tumour of any size that invades any of the following: mediastinum, heart, great vessels, trachea, esophagus, vertebral body, carina; ortumour with a malignant pleural or pericardial effusion; or with separate tumour nodule(s) within the ipsilateral primary-tumour lobe of the lung,
- **N**: **REGIONAL LYMPH NODES**
- NX: Regional lymph nodes cannot be assessed
- N0: No regional lymph node metastasis
- N1: Metastasis in ipsilateral peribronchial and/or ipsilateral hilar lymph nodes and intrapulmonary nodes, including involvement by direct extension
- N2: Metastasis in ipsilateral mediastinal and/or subcarinal lymph node(s)
- N3: Metastasis in contralateral mediastinal, contralateral hilar, ipsilateral or contralateral scalene, or supraclavicular lymph node(s)
- **M**: **DISTANT METASTASIS**
- MX: Distant metastasis cannot be assessed
- M0: No distant metastasis
- M1: Distant metastasis; includes separate tumour nodule(s) in the non- primary-tumour lobe (ipsilateral or contralateral)

| **STAGE GROUPING OF TNM SUBSETS:** | | | |
|---|---|---|---|
| Occult | TX | N0 | M0 |
| carcinoma | | | |
| Stage 0 | Tis | N0 | M0 |
| Stage IA | T1 | N0 | M0 |
| Stage IB | T2 | N0 | M0 |
| Stage IIA | T1 | N1 | M0 |
| Stage IIB | T2 | N1 | M0 |
| | T3 | N0 | M0 |
| Stage IIIA | T3 | N1 | M0 |
| | T1 | N2 | M0 |
| | T2 | N2 | M0 |
| | T3 | N2 | M0 |
| Stage IIIB | Any. T | N3 | M0 |
| | T4 | Any N | M0 |
| Stage IV | Any T | Any N | M1 |

Charts with diagnostic codes 162.9 (lung cancer) and 197 (metastatic cancer) were collected manually and electronically. This information was submitted to the BC Cancer Registry to collect information such as date of diagnosis, date of death, and cancer stage. The charts for those patients treated at the BC Cancer Agency (BCCA) were then requested and reviewed to confirm the date of diagnosis and cancer stage. The information was entered into an Excel spreadsheet created for this purpose. Patients were excluded from the study for the following reasons: 1) wrong stage; 2) missing data; 3) no BCCA chart, or; 4) BCCA chart did not reach in time for the data analysis. 20 patients were excluded from the study because their charts have not arrived yet or there was insufficient information, of which 6 were MRV users. The study group includes 108 patients in total: 50 who took the MRV vaccine and 58 who did not take the MRV vaccine. The researchers applied for and received ethics approval for this study from the University of British Columbia Ethics Review Board, where Dr. Gunn is on Clinical Faculty.

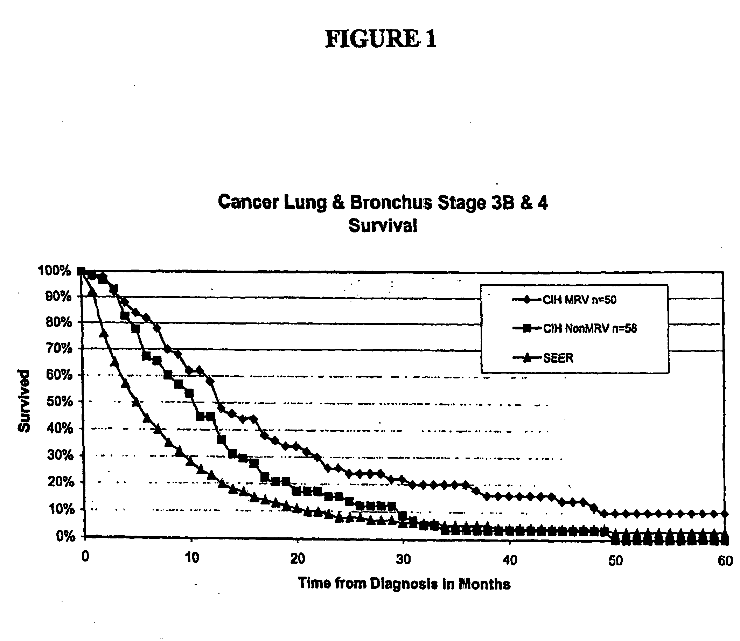

Comparison of survival of patients initially diagnosed with stage 3B and 4 lung cancer who took MRV with patients who didn't take MRV and with SEER standard survival data for patients initially diagnosed with stage 3B and 4 lung cancer (Figure 1) was as follows:

| | SEER | non-MRV | MRV |
|---|---|---|---|
| median survival: | 5 months | 10.5 months | 12.5 months |
| survival at 1 year: | 25% | 45% | 58% |
| survival at 3 years: | 5% | 3% | 20% |
| survival at 5 years: | 3% | 0% | 10% |

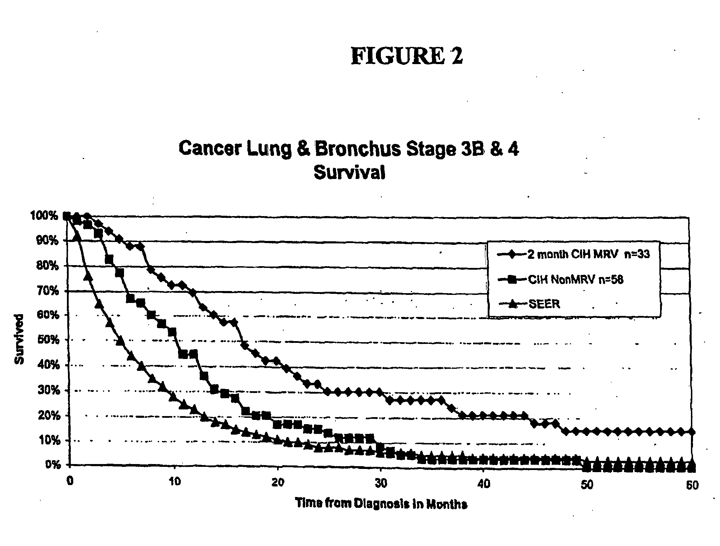

A comparison of survival (as above), including only those patients who took MRV for at least 2 months (Figure 2) is as follows.

| | |
|---|---|
| median survival: | 16.5 months |
| survival at 1 year: | 70% |
| survival at 3 years: | 27% |
| survival at 5 years: | 15% |

Median survival and survival at 1 year, 3 years and 5 years, was substantially better in the group that was treated with MRV (containing bacteria which commonly cause lung infection), evidence of the effectiveness of this vaccine for the treatment of lung cancer. Patients who were treated with the MRV vaccine for more than 2 months had the highest survival rates, further evidence of the effectiveness of this vaccine for the treatment of lung cancer.

### Malignant Melanoma

I.N. (b.d. 1924) was diagnosed with metastatic spread of previously diagnosed malignant melanoma, with two very large metastases to the liver. The prognosis for this type and stage of cancer (stage 4 malignant melanoma, distant spread) is very poor, based on the SEER survival curve. I.N. declined chemotherapy since the benefits of conventional treatment for this type of cancer are very limited. Laparoscopy demonstrated wide-spread peritoneal metastases in addition to the large liver metastases; biopsy was positive for malignant melanoma, and thus, it was determined that his condition was inoperable. I.N. began MRV six weeks after diagnosis of metastatic disease.

I.N. continues to do well 6.5 years after being diagnosed with metastatic malignant melanoma with multiple large liver metastases. He has continued to take the MRV on a regular basis (1-3 times per week) since that time (i.e., 6.5 years), as well as multiple vitamin/supplement regimes, healthful diet and other immune enhancement treatments. In spite of the very poor initial prognosis, now, 6.5 years after being diagnosed with metastatic malignant melanoma, I.N. feels remarkably well. His energy and appetite are excellent, with stable weight, no nausea, and no pain. He bicycles on a daily basis and maintains a very active life. He has an ultrasound of his abdomen every 3 months, and although the large liver metastases have increased in size during some 3 month intervals , his condition has, overall, been remarkably stable considering the length of time since his diagnosis of metastatic disease and the usual prognosis for this illness. Although, his recent abdominal ultrasound showed slight progression of a few of the liver metastases, on the whole, there was little change in the majority of the metastases. His liver function tests remain normal or near normal and he remains in excellent health in spite of the large liver metastases. This 81 year-old patient maintains a very active life and has been remarkably healthy considering the length of time since his diagnosis with terminal cancer and the usual prognosis for this type and stage of cancer.

*Staphylococcus aureus,* one of the most common causes of skin infection, is a major component of all three of the vaccines that I.N. used (see above). In addition, the Polyvaccinum Forte, contains another bacterium which commonly causes skin infection, *Streptococcus pyogenes.* Thus, vaccines including pathogenic bacteria that cause skin infection were effective in treating advanced malignant melanoma, which is skin cancer that is usually quickly fatal.

### Follicular Non-Hodgkin's lymphoma

D.W. (b.d. 1945) was diagnosed with stage 4A Follicular Non-Hodgkin's lymphoma, with extensive marked lymphadenopathy (i.e., enlarged lymph glands). He declined all conventional treatment. D. W. began treatment with the Bayer MRV vaccine, as well as the multiple vitamin/supplement regimes, healthful diet and other immune enhancement treatments. He continued regular use of this vaccine for more than 3 years., at which time his lymph glands had begun to greatly reduce in size and he was feeling well. This resolution of lymphadenopathy continued, and imaging showed almost complete resolution of previous extensive lymphadenopathy. D. W. was feeling well and there was no lymphadenopathy palpable: a clearly remarkable recovery. Five years after his initial diagnosis with Stage 4A Follicular Non-Hodgkin's lymphoma, D.W. had no evidence of recurrence and was leading an active and healthy life. Treatment with the MRV vaccine resulted in complete remission of his stage 4A follicular non-Hodgkins' lymphoma.

### Colon Cancer

S.C. (b.d. 1942) was diagnosed with metastatic spread of previously treated colon cancer, with a metastasis to the liver and probable other metastases to both kidneys. The liver metastasis was excised. The prognosis for this stage (i.e., stage 4) of colon cancer is poor and the benefit of further conventional treatment (i.e., chemotherapy) is limited. S.C. declined chemotherapy initially. Three months after diagnosis with metastatic colon cancer, S.C. began treatment with Polyvaccinum Forte, as well as a multiple vitamin/supplement regime and healthful diet. He continued regular use of this vaccine and the vitamin and supplement regime, and began chemotherapy. Although the overall course of his disease has been slowly progressive, with development of lung metastases and recurrence of liver metastases, 28 months after his initial diagnosis of metastatic disease, his weight was stable and his energy levels were good. Three years (36 months) after diagnosis of stage 4 colon cancer, S.C. was feeling well except for nausea and mild weight loss related to chemotherapy. In spite of a rising tumour marker, he continued to feel well and was continuing to work full-time.

The standard SEER survival data for metastatic colon cancer Colon & Rectum Stage 4, Survival, distant spread, indicate that at 28 months after diagnosis of stage 4 colon cancer, only 16% of patients are still alive. Based on clinical experience, a composition that contains *E. coli* has improved the clinical course and, thus, slowed the progression of the disease.

### Kidney Cancer

R.N. (b.d. 1940), who had been previously diagnosed with kidney cancer with metastases to the bone and lungs, was found to have a brain metastasis. His prognosis was very bleak, in spite of the fact that the brain metastasis was a single lesion that was operable. He had neurosurgery to excise the brain metastasis and began on the Polyvaccinum Forte vaccine. On his most recent follow-up appointment, six months after he was diagnosed with recurrent metastatic disease, he was feeling very well, had good energy and was symptom free. There is no standard survival data for recurrent metastatic kidney cancer with metastasis to the brain, but, as discussed above, the prognosis is very bleak. Based on our clinical experience, it appears as though Polyvaccinum Forte has improved the clinical course in this case. R.N.'s survival and current good health is unexpected and indicates the value of the use of a bacterial vaccine containing *E. coli* for the treatment of kidney cancer. In spite of a very bleak prognosis, R.N. is still living 18 months after being diagnosed with a brain metastasis that was surgically excised and reports feeling very well and healthy. After having taken the bacterial vaccine containing E. coli for just over one year, R.N. discontinued treatment. Subsequently (about 4 months later), he developed mild nausea and headache and was diagnosed with another brain metastasis, possibly a recurrence, at the site of the original metastasis. He has begun treatment with the vaccine containing E. coli again and will have surgery once again.

Accordingly, based on clinical experience, the Bayer and Stallergenes vaccines were effective in improving the course of lung cancer, lymphoma, and malignant melanoma as they including bacteria that were pathogenic in lung, skin, and lymph nodes, but were not as effective in treating, for example, colon cancer as none of the bacteria in these vaccines were pathogenic in the colon. On the other hand, Polyvaccinum Forte includes *E. coli,* which is pathogenic in colon, kidney, bladder, prostate, uterus, and ovaries, and was effective in treating colon, ovarian, kidney, and prostate cancers.

### Colon Cancer

T.B. (b.d. 1935) was diagnosed with metastatic colon cancer with metastases to the liver, porta hepatic lymph nodes and lungs. The prognosis for this stage (i.e., stage 4) of colon cancer is very poor (i.e., 'terminal' cancer) and the benefit of conventional treatment (i.e., chemotherapy) is limited. T.B. began chemotherapy, but discontinued treatment approximately 5 months after his diagnosis due to side effects, at which time he began treatment with Polyvaccinum Forte (containing *E. coli*) every second day as well as a multiple vitamin/supplement regime and a healthy diet.

Polyvaccinum Forte contains *E. coli,* which is the most common cause of infection in the colon and abdominal lymphatic system (e.g. porta hepatic lymph nodes), as well as *Streptococcus pneumoniae, Moraxella catarrhalis, Klebsiella pneumoniae* and *Haemophilus influenzae,* which are the most common causes of infection in the lungs. T.B's most recent CT Scans demonstrate necrotic porta hepatic lymph nodes unchanged in size from the time of his diagnosis and no change in size of the lung metastases, although the two liver metastases grew moderately in size (3.4 cm to 4.5 cm and 1.2 cm to 3.0 cm). In spite of the very poor prognosis, T.B. has continued to feel quite well almost one year after a diagnosis of terminal cancer.

### Pancreatic Cancer

A.M. (b.d. 1933) was diagnosed with pancreatic cancer, at which time he had surgery to remove his pancreas (i.e., Whipple's procedure). However, he developed metastases to the lungs bilaterally approximately a year after his diagnosis of pancreatic cancer. The diagnosis of lung metastases is a terminal diagnosis with very poor prognosis. A.M. has taken palliative chemotherapy intermittently over the last three years and began Polyvaccinum Forte a little under three years after his diagnosis of pancreatic cancer. Polyvaccinum Forte contains *E. coli,* which is the most common cause of infection in the region of the pancreas, as well as *Streptococcus pneumoniae, Moraxella catarrhalis, Klebsiella pneumoniae* and *Haemophilus influenzae,* which are the most common causes of infection in the lungs. In spite of a very poor prognosis, A.M. continues to feel remarkably well considering it is almost three years since he was diagnosed with recurrent metastatic pancreatic cancer, which has an extremely poor prognosis.

Although various embodiments of the invention are disclosed herein, many adaptations and modifications may be made in accordance with the common general knowledge of those skilled in this art. Such modifications include the substitution of known equivalents for any aspect of the invention in order to achieve the same result in substantially the same way. Numeric ranges are inclusive of the numbers defining the range. In the specification, the word "comprising" is used as an open-ended term, substantially equivalent to the phrase "including, but not limited to", and the word "comprises" has a corresponding meaning. Citation of references herein shall not be construed as an admission that such references are prior art to the present invention.

## Claims

1. A preparation of an antigen of one or more pathogenic bacterial species for use in treating a cancer in a specific organ, tissue or cell in a subject, wherein the preparation elicits an immune response in the subject, and wherein the pathogenic bacterial species is pathogenic in the corresponding specific organ, tissue or cell in a healthy subject, and wherein the antigen of one or more pathogenic bacterial species is provided as one or more attenuated bacteria, and wherein:
the cancer is situated in the kidney and the one or more pathogenic bacterial species are selected from *Escherichia coli, Proteus mirabilis, Proteus vulgatus, Providentia* species, *Morganella* species and *Enterococcus faecalis;* or
the cancer is situated in the lung and the one or more pathogenic bacterial species are selected from *Streptococcus pneumoniae, Moraxella catarrhalis, Mycoplasma pneumoniae, Klebsiella pneumoniae, Haemophilus influenzae, Staphylococcus aureus, Chlamydia pneumoniae and Legionella pneumophila;* or
the cancer is situated in the bone and the pathogenic bacterial species is *Staphylococcus aureus;* or
the cancer is situated in the colon and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* and *Shigella flexneri;* or
the cancer is situated in the prostate and the one or more pathogenic bacterial species are selected from *Escherichia coli, Corynebacterium* species, *Enterococcus faecalis* and *Neisseria gonorrhoeae;* or
the cancer is situated in the skin and the one or more pathogenic bacterial species are selected from *Staphylococcus aureus, Corynebacterium diphtheriae, Corynebacterium ulcerans* and *Pseudomonas aeruginosa;* or
the cancer is situated in the mouth and the one or more pathogenic bacterial species are selected from *Prevotella melaninogenicus,* anaerobic streptococci, viridans streptococci and *Actinomyces* species; or
the cancer is situated in the testicle and the one or more pathogenic bacterial species are selected from *Escherichia coli, Salmonella enteritidis* and *Staphyloccocus aureus;* or
the cancer is situated in the uterus and the one or more pathogenic bacterial species are selected from *Bacteroides fragilis, Escherichia coli, Neisseria gonorrhoeae* and *Chlamydie trachomatis;* or
the cancer is situated in the ovary and the one or more pathogenic bacterial species are selected from *Bacteroides fragilis, Escherichia coli, Neisseria gonorrhoeae* and *Chlamydia trachomatis;* or
the cancer is situated in the vagina and the one or more pathogenic bacterial species are selected from *Bacteroides fragilis* and *Escherichia coli;* or
the cancer is situated in the breast and the pathogenic bacterial species is *Staphyloccocus aureus;* or
the cancer is situated in the gallbladder and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* and *Shigella flexneri;* or
the cancer is situated in the bladder and the pathogenic bacterial species is *Escherichia coli;* or
the cancer is lymphoma associated with the head or neck and the one or more pathogenic bacterial species are selected from *Corynebacterium diptheriae, Corynebacterium ulcerans, Arcanobacterium haemolyticum, Staphylococcus aureus, Pseudomonas aeruginosa, Prevotella melaninogenicus,* anaerobic streptococci, viridans streptococci and *Actinomyces* species; or
the cancer is lymphoma associated with the chest and the one or more pathogenic bacterial species are selected from *Streptococcus pneumoniae, Moraxella catarrhalis, Mycoplasma pneumoniae, Klebsiella pneumoniae, Haemophilus influenzae, Staphylococcus aureus, Chlamydia pneumoniae* and *Legionella pneumophila;* or
the cancer is lymphoma associated with the abdominal cavity and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica, Shigella flexneri, Proteus mirabilis, Proteus vulgatus, Providentia* species, *Morganella* species and *Enterococcus faecalis;* or
the cancer is lymphoma associated with the axilliary or inguinal area and the one ore more pathogenic bacterial species are selected from *Staphylococcus aureus, Corynebacterium diphtheriae, Corynebacterium ulcerans* and *Pseudomonas aeruginosa.*

2. Use of a preparation of an antigen of one or more pathogenic bacterial species in the manufacture of a medicament for treating a cancer in a specific organ, tissue or cell in a subject, wherein the preparation elicits an immune response in the subject, and wherein the pathogenic bacterial species is pathogenic in the corresponding specific organ, tissue or cell in a healthy subject, and wherein the antigen of one or more pathogenic bacterial species is provided as one or more attenuated bacteria, and wherein:
the cancer is situated in the kidney and the one or more pathogenic bacterial species are selected from *Escherichia coli, Proteus mirabilis, Proteus vulgatus, Providentia* species, *Morganella* species and *Enterococcus faecalis;* or
the cancer is situated in the lung and the one or more pathogenic bacterial species are selected from *Streptococcus pneumoniae, Moraxella catarrhalis, Mycoplasma pneumoniae, Klebsiella pneumoniae, Haemophilus influenzae, Staphylococcus aureus, Chlamydia pneumoniae and Legionella pneumophila;* or
the cancer is situated in the bone and the pathogenic bacterial species is *Staphylococcus aureus;* or
the cancer is situated in the colon and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* and *Shigella flexneri;* or
the cancer is situated in the prostate and the one or more pathogenic bacterial species are selected from *Escherichia coli, Corynebacterium* species, *Enterococcus faecalis* and *Neisseria gonorrhoeae;* or
the cancer is situated in the skin and the one or more pathogenic bacterial species are selected from *Staphylococcus aureus, Corynebacterium diphtheriae, Corynebacterium ulcerans* and *Pseudomonas aeruginosa;* or
the cancer is situated in the mouth and the one or more pathogenic bacterial species are selected from *Prevotella melaninogenicus,* anaerobic streptococci, viridans streptococci and *Actinomyces* species; or
the cancer is situated in the testicle and the one or more pathogenic bacterial species are selected from *Escherichia coli, Salmonella enteritidis* and *Staphyloccocus aureus;* or
the cancer is situated in the uterus and the one or more pathogenic bacterial species are selected from *Bacteroides fragilis, Escherichia coli, Neisseria gonorrhoeae* and *Chlamydia trachomatis;* or
the cancer is situated in the ovary and the one or more pathogenic bacterial species are selected from *Bacteroides fragilis, Escherichia coli, Neisseria gonorrhoeae* and *Chlamydia trachomatis;* or
the cancer is situated in the vagina and the one or more pathogenic bacterial species are selected from *Bacteroides fragilis* and *Escherichia coli;* or
the cancer is situated in the breast and the pathogenic bacterial species is *Staphyloccocus aureus;* or
the cancer is situated in the gallbladder and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* and *Shigella flexneri;* or
the cancer is situated in the bladder and the pathogenic bacterial species is *Escherichia coli;* or
the cancer is lymphoma associated with the head or neck and the one or more pathogenic bacterial species are selected from *Corynebacterium diptheriae, Corynebacterium ulcerans, Arcanobacterium haemolyticum, Staphylococcus aureus, Pseudomonas aeruginosa, Prevotella melaninogenicus,* anaerobic streptococci, viridans streptococci and *Actinomyces* species; or
the cancer is lymphoma associated with the chest and the one or more pathogenic bacterial species are selected from *Streptococcus pneumoniae, Moraxella catarrhalis, Mycoplasma pneumoniae, Klebsiella pneumoniae, Haemophilus influenzae, Staphylococcus aureus, Chlamydia pneumoniae* and *Legionella pneumophila;* or
the cancer is lymphoma associated with the abdominal cavity and the one or more pathogenic bacterial species are selected from *Bacteroides fragilis, Bacteroides vulgatus, Bacteroides thetaiotaomicron, Clostridium perfringens, Clostridium difficile, Escherichia coli, Salmonella enteritidis, Yersinia enterocolitica, Shigella flexneri, Proteus mirabilis, Proteus vulgatus, Providentia* species, *Morganella* species and *Enterococcus faecalis;* or
the cancer is lymphoma associated with the axilliary or inguinal area and the one ore more pathogenic bacterial species are selected from *Staphylococcus aureus, Corynebacterium diphtheriae, Corynebacterium ulcerans* and *Pseudomonas aeruginosa.*

3. The preparation for use according to Claim 1 or the use of Claim 2, wherein said cancer is lung cancer and the pathogenic bacterial species are *Streptococcus pneumoniae, Moraxella catarrhalis* or *Mycoplasma pneumoniae.*

4. A preparation for use according to Claim 1 or the use of Claim 2 wherein said cancer is lung cancer, and said pathogenic - bacterial species are *Streptococcus pneumoniae, Moraxella catarrhalis, Klebsiella pneumoniae, Haemophilus influenzae,* or *Staphylococcus aureus.*

5. The preparation for use according to Claim 1 or the use of Claim 2, wherein said cancer is mouth cancer and the pathogenic bacterial species are viridans streptococci selected from *Streptococcus salivarius, Streptococcus sanguis,* and *Streptococcus mutans.*

6. The preparation for use according to Claim 1 or the use of Claim 2, wherein said cancer is gallbladder cancer and the pathogenic bacterial species is *Escherichia coli.*

7. A preparation for use according to any preceding claim or the use according to any preceding claim, wherein said pathogenic bacterial species is capable of causing infection when present in said specific organ, tissue or cell in a healthy subject.

8. A preparation for use according to any preceding claim or the use according to any preceding claim, wherein said pathogenic bacterial species has caused an infection in said specific organ, tissue or cell in a healthy subject.

9. A preparation for use according to any preceding claim or the use according to any preceding claim, wherein said antigen is for administration by administering a whole bacterial species.

10. A preparation for use according to any preceding claim or the use according to any preceding claim, wherein the subject is to be administered at least two or more bacterial species.

11. A preparation for use according to Claim 10 or the use according to Claim 10, wherein the subject is to be administered at least three or more bacterial species.

12. A preparation for use according to any preceding claim or the use according to any preceding claim, wherein the subject is to be administered a supplement.

13. A preparation for use according to any preceding claim or the use according to any preceding claim, wherein the subject is to be administered an adjuvant.

14. A preparation for use according to Claims 1 or the use according to Claim 2, wherein the preparation is for subcutaneous administration.

15. A preparation for use according to Claim 14, or the use according to Claim 14, wherein the preparation is for administration at a dose sufficient to cause a local skin reaction, such as a one to three inch (2.54 cm to 7.62 cm) diameter round patch of pinkness or redness at the injection site.

16. A preparation for use according to Claim 1 or the use according to Claim 2, wherein the preparation is for administration as several divided doses over time, such as for administration every second day or three times per week.

17. A preparation for use according to Claim 16, or the use according to Claim 16, wherein the preparation is for administration three times per week and the doses are administered for a period of at least six months.

18. A preparation for use according to Claim 17, or the use according to Claim 17, wherein the preparation is for subcutaneous administration at a dose sufficient to cause a local skin reaction, such as a one to three inch (2.54 cm to 7.62 cm) diameter round patch of pinkness or redness at the injection site, preferably a one to two inch (2.54 cm to 5.08 cm) diameter round patch of pinkness or redness at the injection site.

## Patentansprüche

1. Präparat eines Antigens von einer oder mehreren pathogenen Bakterien-Spezies für die Verwendung bei der Behandlung von Krebs in einem bestimmten Organ, Gewebe oder einer bestimmten Zelle eines Subjekts, wobei das Präparat eine Immunreaktion in dem Subjekt bewirkt und wobei die pathogene Bakterien-Spezies in dem entsprechenden bestimmten Organ, Gewebe oder der bestimmten Zelle eines gesunden Subjekts pathogen ist und wobei das Antigen von einer oder mehreren pathogenen Bakterien-Spezies in Form eines attenuierten Bakteriums oder mehrerer attenuierter Bakterien bereitgestellt wird und wobei:
der Krebs in der Niere lokalisiert ist und die eine oder die mehreren pathogene(n) Bakterien-Spezies aus *Escherichia coli, Proteus mirabilis, Proteus vulgatus, Providentia-*Spezies, Morganella-Spezies und *Enterococcus faecalis* ausgewählt ist bzw. sind, oder
der Krebs in der Lunge lokalisiert ist und die eine oder die mehreren pathogene(n) Bakterien-Spezies aus *Streptococcus pneumoniae, Moraxella catarrhalis, Mycoplasma pneumoniae, Klebsiella pneumoniae, Haemophilus influenzae, Staphylococcus aureus, Chlamydia pneumoniae* und *Legionella pneumophila* ausgewählt ist bzw. sind, oder
der Krebs im Knochen lokalisiert ist und die pathogene Bakterien-Spezies *Staphylococcus aureus* ist, oder
der Krebs im Kolon lokalisiert ist und die eine oder die mehreren pathogene(n) Bakterien-Spezies aus *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* und *Shigella flexneri* ausgewählt ist bzw. sind, oder
der Krebs in der Prostata lokalisiert ist und die eine oder die mehreren pathogene(n) Bakterien-Spezies aus *Escherichia coli, Corynebacterium-Spezies, Enterococcus faecalis* und *Neisseria gonorrhoeae* ausgewählt ist bzw. sind, oder
der Krebs in der Haut lokalisiert ist und die eine oder die mehreren pathogene(n) Bakterien-Spezies aus *Staphylococcus aureus, Corynebacterium diphtheriae, Corynebacterium ulcerans* und *Pseudomonas aeruginosa* ausgewählt ist bzw. sind, oder
der Krebs im Mund lokalisiert ist und die eine oder die mehreren pathogene(n) Bakterien-Spezies aus *Prevotella melaninogenicus, anaeroben* Streptokokken, Viridans-Streptokokken und *Actinomyces-Spezies* ausgewählt ist bzw. sind, oder
der Krebs im Hoden lokalisiert ist und die eine oder die mehreren pathogene(n) Bakterien-Spezies aus *Escherichia coli, Salmonella enteritidis* und *Staphylococcus aureus* ausgewählt ist bzw. sind, oder
der Krebs im Uterus lokalisiert ist und die eine oder die mehreren pathogene(n) Bakterien-Spezies aus *Bacteroides fragilis, Escherichia coli, Neisseria gonorrhoeae* und *Chlamydia trachomatis* ausgewählt ist bzw. sind, oder
der Krebs im Ovar lokalisiert ist und die eine oder die mehreren pathogene(n) Bakterien-Spezies aus *Bacteroides fragilis, Escherichia coli, Neisseria gonorrhoeae* und *Chlamydia trachomatis* ausgewählt ist bzw. sind, oder
der Krebs in der Vagina lokalisiert ist und die eine oder die mehreren pathogene(n) Bakterien-Spezies aus *Bacteroides fragilis* und *Escherichia coli* ausgewählt ist bzw. sind, oder
der Krebs in der Brust lokalisiert ist und die pathogene Bakterien-Spezies *Staphylococcus aureus* ist, oder
der Krebs in der Gallenblase lokalisiert ist und die eine oder die mehreren pathogene(n) Bakterien-Spezies aus *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* und *Shigella flexneri* ausgewählt ist bzw. sind, oder
der Krebs in der Blase lokalisiert ist und die pathogene Bakterien-Spezies *Escherichia coli* ist, oder
der Krebs ein Lymphom ist, das mit dem Kopf oder Hals assoziiert ist, und die eine oder die mehreren pathogene(n) Bakterien-Spezies aus *Corynebacterium diphtheriae, Corynebacterium ulcerans, Arcanobacterium haemolyticum, Staphylococcus aureus, Pseudomonas aeruginosa, Prevotella melaninogenicus, anaeroben* Streptokokken, Viridans-Streptokokken und *Actinomyces-Spezies* ausgewählt ist bzw. sind, oder
der Krebs ein Lymphom ist, das mit dem Brustkorb assoziiert ist, und die eine oder die mehreren pathogene(n) Bakterien-Spezies aus *Streptococcus pneumoniae, Moraxella catarrhalis, Mycoplasma pneumoniae, Klebsiella pneumoniae, Haemophilus influenzae, Staphylococcus aureus, Chlamydia pneumoniae* und *Legionella pneumophila* ausgewählt ist bzw. sind, oder
der Krebs ein Lymphom ist, das mit der Bauchhöhle assoziiert ist, und die eine oder die mehreren pathogene(n) Bakterien-Spezies aus *Bacteroides fragilis, Bacteroides vulgatus, Bacteroides thetaiotaomicron, Clostridium perfringens, Clostridium difficile, Escherichia coli, Salmonella enteritidis, Yersinia enterocolitica, Shigella flexneri, Proteus mirabilis, Proteus vulgatus, Providentia-Spezies,* Morganella-Spezies und *Enterococcus faecalis* ausgewählt ist bzw. sind, oder
der Krebs ein Lymphom ist, das mit der Achselhöhle oder der Leistengegend assoziiert ist, und die eine oder die mehreren pathogene(n) Bakterien-Spezies aus *Staphylococcus aureus, Corynebacterium diphtheriae, Corynebacterium ulcerans* und *Pseudomonas aeruginosa* ausgewählt ist bzw. sind.

2. Verwendung eines Präparats eines Antigens von einer oder mehreren pathogenen Bakterien-Spezies bei der Herstellung eines Medikaments für die Behandlung von Krebs in einem bestimmten Organ, Gewebe oder einer bestimmten Zelle eines Subjekts, wobei das Präparat eine Immunreaktion in dem Subjekt bewirkt und wobei die pathogene Bakterien-Spezies in dem entsprechenden bestimmten Organ, Gewebe oder der bestimmten Zelle eines gesunden Subjekts pathogen ist und wobei das Antigen von einer oder mehreren pathogenen Bakterien-Spezies in Form eines attenuierten Bakteriums oder mehrerer attenuierter Bakterien bereitgestellt wird und wobei:
der Krebs in der Niere lokalisiert ist und die eine oder die mehreren pathogene(n) Bakterien-Spezies aus *Escherichia coli, Proteus mirabilis, Proteus vulgatus, Providentia-*Spezies, Morganella-Spezies und *Enterococcus faecalis* ausgewählt ist bzw. sind, oder
der Krebs in der Lunge lokalisiert ist und die eine oder die mehreren pathogene(n) Bakterien-Spezies aus *Streptococcus pneumoniae, Moraxella catarrhalis, Mycoplasma pneumoniae, Klebsiella pneumoniae, Haemophilus influenzae, Staphylococcus aureus, Chlamydia pneumoniae* und *Legionella pneumophila* ausgewählt ist bzw. sind, oder
der Krebs im Knochen lokalisiert ist und die pathogene Bakterien-Spezies *Staphylococcus aureus* ist, oder
der Krebs im Kolon lokalisiert ist und die eine oder die mehreren pathogene(n) Bakterien-Spezies aus *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* und *Shigella flexneri* ausgewählt ist bzw. sind, oder
der Krebs in der Prostata lokalisiert ist und die eine oder die mehreren pathogene(n) Bakterien-Spezies aus *Escherichia coli, Corynebacterium-Spezies, Enterococcus faecalis* und *Neisseria gonorrhoeae* ausgewählt ist bzw. sind, oder
der Krebs in der Haut lokalisiert ist und die eine oder die mehreren pathogene(n) Bakterien-Spezies aus *Staphylococcus aureus, Corynebacterium diphtheriae, Corynebacterium ulcerans* und *Pseudomonas aeruginosa* ausgewählt ist bzw. sind, oder
der Krebs im Mund lokalisiert ist und die eine oder die mehreren pathogene(n) Bakterien-Spezies aus *Prevotella melaninogenicus, anaeroben* Streptokokken, Viridans-Streptokokken und *Actinomyces-Spezies* ausgewählt ist bzw. sind, oder
der Krebs im Hoden lokalisiert ist und die eine oder die mehreren pathogene(n) Bakterien-Spezies aus *Escherichia coli, Salmonella enteritidis* und *Staphylococcus aureus* ausgewählt ist bzw. sind, oder
der Krebs im Uterus lokalisiert ist und die eine oder die mehreren pathogene(n) Bakterien-Spezies aus *Bacteroides fragilis, Escherichia coli, Neisseria gonorrhoeae* und *Chlamydia trachomatis* ausgewählt ist bzw. sind, oder
der Krebs im Ovar lokalisiert ist und die eine oder die mehreren pathogene(n) Bakterien-Spezies aus *Bacteroides fragilis, Escherichia coli, Neisseria gonorrhoeae* und *Chlamydia trachomatis* ausgewählt ist bzw. sind, oder
der Krebs in der Vagina lokalisiert ist und die eine oder die mehreren pathogene(n) Bakterien-Spezies aus *Bacteroides fragilis* und *Escherichia coli* ausgewählt ist bzw. sind, oder
der Krebs in der Brust lokalisiert ist und die pathogene Bakterien-Spezies *Staphylococcus aureus* ist, oder
der Krebs in der Gallenblase lokalisiert ist und die eine oder die mehreren pathogene(n) Bakterien-Spezies aus *Bacteroides fragilis, Bacteroides vulgatus, Bacteroides thetaiotaomicron, Clostridium periringens, Clostridium difficile, Escherichia coli, Salmonella enteritidis, Yersinia enterocolitica* und *Shigella flexneri* ausgewählt ist bzw. sind, oder
der Krebs in der Blase lokalisiert ist und die pathogene Bakterien-Spezies *Escherichia coli* ist, oder
der Krebs ein Lymphom ist, das mit dem Kopf oder Hals assoziiert ist, und die eine oder die mehreren pathogene(n) Bakterien-Spezies aus *Corynebacterium diphtheriae, Corynebacterium ulcerans, Arcanobacterium haemolyticum, Staphylococcus aureus, Pseudomonas aeruginosa, Prevotella melaninogenicus, anaeroben* Streptokokken, Viridans-Streptokokken und *Actinomyces-Spezies* ausgewählt ist bzw. sind, oder
der Krebs ein Lymphom ist, das mit dem Brustkorb assoziiert ist, und die eine oder die mehreren pathogene(n) Bakterien-Spezies aus *Streptococcus pneumoniae, Moraxella catarrhalis, Mycoplasma pneumoniae, Klebsiella pneumoniae, Haemophilus influenzae, Staphylococcus aureus, Chlamydia pneumoniae* und *Legionella pneumophila* ausgewählt ist bzw. sind, oder
der Krebs ein Lymphom ist, das mit der Bauchhöhle assoziiert ist, und die eine oder die mehreren pathogene(n) Bakterien-Spezies aus *Bacteroides fragilis, Bacteroides vulgatus, Bacteroides thetaiotaomicron, Clostridium perfringens, Clostridium difficile, Escherichia coli, Salmonella enteritidis, Yersinia enterocolitica, Shigella flexneri, Proteus mirabilis, Proteus vulgatus, Providentia-Spezies, Morganella*-Spezies und *Enterococcus faecalis* ausgewählt ist bzw. sind, oder
der Krebs ein Lymphom ist, das mit der Achselhöhle oder der Leistengegend assoziiert ist, und die eine oder die mehreren pathogene(n) Bakterien-Spezies aus *Staphylococcus aureus, Corynebacterium diphtheriae, Corynebacterium ulcerans* und *Pseudomonas aeruginosa* ausgewählt ist bzw. sind.

3. Präparat für eine Verwendung gemäß Anspruch 1 oder Verwendung nach Anspruch 2, wobei der Krebs Lungenkrebs ist und die pathogenen Bakterien-Spezies *Streptococcus pneumoniae, Moraxella catarrhalis* oder *Mycoplasma pneumoniae* sind.

4. Präparat für eine Verwendung gemäß Anspruch 1 oder Verwendung nach Anspruch 2, wobei der Krebs Lungenkrebs ist und die pathogenen Bakterien-Spezies *Streptococcus pneumoniae, Moraxella catarrhalis, Klebsiella pneumoniae, Haemophilus influenzae* oder *Staphylococcus aureus* sind.

5. Präparat für eine Verwendung gemäß Anspruch 1 oder Verwendung nach Anspruch 2, wobei der Krebs Mundkrebs ist und die pathogenen Bakterien-Spezies Viridans-Streptokokken sind, die aus *Streptococcus salivarius, Streptococcus sanguis* und *Streptococcus mutans* ausgewählt sind.

6. Präparat für eine Verwendung gemäß Anspruch 1 oder Verwendung nach Anspruch 2, wobei der Krebs Gallenblasenkrebs ist und die pathogene Bakterien-Spezies *Escherichia coli* ist.

7. Präparat für eine Verwendung gemäß irgendeinem vorhergehenden Anspruch oder Verwendung gemäß irgendeinem vorhergehenden Anspruch, wobei die pathogene Bakterien-Spezies in der Lage ist, eine Infektion hervorzurufen, wenn sie in dem bestimmten Organ, Gewebe oder der bestimmten Zelle in einem gesunden Subjekt vorhanden ist.

8. Präparat für eine Verwendung gemäß irgendeinem vorhergehenden Anspruch oder Verwendung gemäß irgendeinem vorhergehenden Anspruch, wobei die pathogene Bakterien-Spezies in dem bestimmten Organ, Gewebe oder der bestimmten Zelle in einem gesunden Subjekt eine Infektion verursacht hat.

9. Präparat für eine Verwendung gemäß irgendeinem vorhergehenden Anspruch oder Verwendung gemäß irgendeinem vorhergehenden Anspruch, wobei das Antigen eines für eine Verabreichung über das Verabreichen einer ganzen Bakterien-Spezies ist.

10. Präparat für eine Verwendung gemäß irgendeinem vorhergehenden Anspruch oder Verwendung gemäß irgendeinem vorhergehenden Anspruch, wobei dem Subjekt wenigstens zwei oder mehr Bakterien-Spezies zu verabreichen sind.

11. Präparat für eine Verwendung gemäß Anspruch 10 oder Verwendung gemäß Anspruch 10, wobei dem Subjekt wenigstens drei oder mehr Bakterien-Spezies zu verabreichen sind.

12. Präparat für eine Verwendung gemäß irgendeinem vorhergehenden Anspruch oder Verwendung gemäß irgendeinem vorhergehenden Anspruch, wobei dem Subjekt ein Ergänzungsmittel zu verabreichen ist.

13. Präparat für eine Verwendung gemäß irgendeinem vorhergehenden Anspruch oder Verwendung gemäß irgendeinem vorhergehenden Anspruch, wobei dem Subjekt ein Adjuvans zu verabreichen ist.

14. Präparat für eine Verwendung gemäß Anspruch 1 oder Verwendung gemäß Anspruch 2, wobei das Präparat eines für eine subkutane Verabreichung ist.

15. Präparat für eine Verwendung gemäß Anspruch 14 oder Verwendung gemäß Anspruch 14, wobei das Präparat eines für eine Verabreichung in einer Dosis ist, die ausreicht, eine lokale Hautreaktion hervorzurufen, wie einen runden pinkfarbenen oder roten Fleck von einem bis drei Inch (2,54 cm bis 7,62 cm) Durchmesser an der Injektionsstelle.

16. Präparat für eine Verwendung gemäß Anspruch 1 oder Verwendung gemäß Anspruch 2, wobei das Präparat eines für eine Verabreichung in Form mehrerer Teildosen über die Zeit, wie für eine Verabreichung alle zwei Tage oder dreimal pro Woche, ist.

17. Präparat für eine Verwendung gemäß Anspruch 16 oder Verwendung gemäß Anspruch 16, wobei das Präparat eines für eine Verabreichung dreimal pro Woche ist und die Dosen über einen Zeitraum von wenigstens sechs Monaten verabreicht werden.

18. Präparat für eine Verwendung gemäß Anspruch 17 oder Verwendung gemäß Anspruch 17, wobei das Präparat eines für die subkutane Verabreichung in einer Dosis ist, die ausreicht, eine lokale Hautreaktion hervorzurufen, wie einen runden pinkfarbenen oder roten Fleck von einem bis drei Inch (2,54 cm bis 7,62 cm) Durchmesser an der Injektionsstelle, vorzugsweise einen runden pinkfarbenen oder roten Fleck von einem bis zwei Inch (2,54 cm bis 5,08 cm) Durchmesser an der Injektionsstelle.

## Revendications

1. Préparation d'un antigène d'une ou de plusieurs espèces bactériennes pathogènes pour une utilisation dans le traitement d'un cancer dans un organe, un tissu ou une cellule spécifique chez un sujet, où la préparation déclenche une réponse immunitaire chez le sujet, et où l'espèce bactérienne pathogène est pathogène dans l'organe, le tissu ou la cellule spécifique correspondant chez un sujet en bonne santé, et où l'antigène d'une ou de plusieurs espèces bactériennes pathogènes est fourni sous la forme d'une ou de plusieurs bactéries atténuées, et où :
le cancer est situé dans le rein et la ou les plusieurs espèces bactériennes pathogènes sont choisies parmi *Escherichia coli*, *Proteus mirabilis, Proteus vulgatus,* l'espèce *Providentia,* l'espèce *Morganella* et *Enterococcus faecalis ;* ou
le cancer est situé dans le poumon et la ou les plusieurs espèces bactériennes pathogènes sont choisies parmi *Streptococcus pneumoniae, Moraxella catarrhalis, Mycoplasma pneumoniae, Klebsiella pneumoniae, Haemophilus influenzae, Staphylococcus aureus, Chlamydia pneumoniae* et *Legionella pneumophila ;* ou
le cancer est situé dans les os et l'espèce bactérienne pathogène est *Staphylococcus aureus ;* ou
le cancer est situé dans le côlon et la ou les plusieurs espèces bactériennes pathogènes sont choisies parmi *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* et *Shigella flexneri ;* ou
le cancer est situé dans la prostate et la ou les plusieurs espèces bactériennes pathogènes sont choisies parmi *Escherichia coli,* l'espèce *Corynebacterium, Enterococcus faecalis* et *Neisseria gonorrhoeae ;* ou
le cancer est situé dans la peau et la ou les plusieurs espèces bactériennes pathogènes sont choisies parmi *Staphylococcus aureus, Corynebacterium diphtheriae, Corynebacterium ulcerans* et *Pseudomonas aeruginosa ;* ou
le cancer est situé dans la bouche et la ou les plusieurs espèces bactériennes pathogènes sont choisies parmi *Prevotella melaninogenicus,* les streptocoques anaérobies, les streptocoques viridans et l'espèce *Actinomyces ;* ou
le cancer est situé dans le testicule et la ou les plusieurs espèces bactériennes pathogènes sont choisies parmi *Escherichia coli, Salmonella enteritidis* et *Staphylococcus aureus ;* ou
le cancer est situé dans l'utérus et la ou les plusieurs espèces bactériennes pathogènes sont choisies parmi *Bacteroides fragilis, Escherichia coli, Neisseria gonorrhoeae* et *Chlamydia trachomatis ;* ou
le cancer est situé dans l'ovaire et la ou les plusieurs espèces bactériennes pathogènes sont choisies parmi *Bacteroides fragilis, Escherichia* coli, *Neisseria gonorrhoeae* et *Chlamydia trachomatis ;* ou
le cancer est situé dans le vagin et la ou les plusieurs espèces bactériennes pathogènes sont choisies parmi *Bacteroides fragilis* et *Escherichia coli* ; ou
le cancer est situé dans le sein et l'espèce bactérienne pathogène est *Staphylococcus aureus ;* ou
le cancer est situé dans la vésicule biliaire et la ou les plusieurs espèces bactériennes pathogènes sont choisies parmi *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* et *Shigella flexneri ;* ou
le cancer est situé dans la vessie et l'espèce bactérienne pathogène est *Escherichia coli ;* ou
le cancer est un lymphome associé à la tête ou au cou et la ou les plusieurs espèces bactériennes pathogènes sont choisies parmi *Corynebacterium diphtheriae, Corynebacterium ulcerans, Arcanobacterium haemolyticum, Staphylococcus aureus, Pseudomonas aeruginosa, Prevotella melaninogeni*cus, les streptocoques anaérobies, les streptocoques viridans et l'espèce *Actinomyces ;* ou
le cancer est un lymphome associé au thorax et la ou les plusieurs espèces bactériennes pathogènes sont choisies parmi *Streptococcus pneumoniae, Moraxella catarrhalis, Mycoplasma pneumoniae, Klebsiella pneumoniae, Haemophilus influenzae, Staphylococcus aureus, Chlamydia pneumoniae* et *Legionella pneumophila ;* ou
le cancer est un lymphome associé à la cavité abdominale et la ou les plusieurs espèces bactériennes pathogènes sont choisies parmi *Bacteroides fragilis, Bacteroides vulgatus, Bacteroides thetaiotaomicron, Clostridium perfringens, Clostridium difficile, Escherichia coli, Salmonella enteritidis, Yersinia enterocolitica, Shigella flexneri, Proteus mirabilis, Proteus vulgatus,* l'espèce *Providentia,* l'espèce *Morganella* et *Enterococcus faecalis ;* ou
le cancer est un lymphome associé aux zones axillaires ou inguinales et la ou les plusieurs espèces bactériennes pathogènes sont choisies parmi *Staphylococcus aureus, Corynebacterium diphtheriae, Corynebacterium ulcerans* et *Pseudomonas aeruginosa.*

2. Utilisation d'une préparation d'un antigène d'une ou de plusieurs espèces bactériennes pathogènes dans la fabrication d'un médicament destiné au traitement d'un cancer dans un organe, un tissu ou une cellule spécifique chez un sujet, où la préparation déclenche une réponse immunitaire chez le sujet, et où l'espèce bactérienne pathogène est pathogène dans l'organe, le tissu ou la cellule spécifique correspondant chez un sujet en bonne santé, et où l'antigène d'une ou de plusieurs espèces bactériennes pathogènes est fourni sous la forme d'une ou de plusieurs bactéries atténuées, et où :
le cancer est situé dans le rein et la ou les plusieurs espèces bactériennes pathogènes sont choisies parmi *Escherichia coli*, *Proteus mirabilis, Proteus vulgatus,* l'espèce *Providentia,* l'espèce *Morganella* et *Enterococcus faecalis ;* ou
le cancer est situé dans le poumon et la ou les plusieurs espèces bactériennes pathogènes sont choisies parmi *Streptococcus pneumoniae, Moraxella catarrhalis, Mycoplasma pneumoniae, Klebsiella pneumoniae, Haemophilus influenzae, Staphylococcus aureus, Chlamydia pneumoniae* et *Legionella pneumophila ;* ou
le cancer est situé dans les os et l'espèce bactérienne pathogène est *Staphylococcus aureus ;* ou
le cancer est situé dans le côlon et la ou les plusieurs espèces bactériennes pathogènes sont choisies parmi *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* et *Shigella flexneri ;* ou
le cancer est situé dans la prostate et la ou les plusieurs espèces bactériennes pathogènes sont choisies parmi *Escherichia coli,* l'espèce *Corynebacterium, Enterococcus faecalis* et *Neisseria gonorrhoeae ;* ou
le cancer est situé dans la peau et la ou les plusieurs espèces bactériennes pathogènes sont choisies parmi *Staphylococcus aureus, Corynebacterium diphtheriae, Corynebacterium ulcerans* et *Pseudomonas aeruginosa ;* ou
le cancer est situé dans la bouche et la ou les plusieurs espèces bactériennes pathogènes sont choisies parmi *Prevotella melaninogenicus,* les streptocoques anaérobies, les streptocoques viridans et l'espèce *Actinomyces ;* ou
le cancer est situé dans le testicule et la ou les plusieurs espèces bactériennes pathogènes sont choisies parmi *Escherichia coli*, *Salmonella enteritidis* et *Staphylococcus aureus ;* ou
le cancer est situé dans l'utérus et la ou les plusieurs espèces bactériennes pathogènes sont choisies parmi *Bacteroides fragilis, Escherichia coli, Neisseria gonorrhoeae* et *Chlamydia trachomatis ;* ou
le cancer est situé dans l'ovaire et la ou les plusieurs espèces bactériennes pathogènes sont choisies parmi *Bacteroides fragilis, Escherichia coli, Neisseria gonorrhoeae* et *Chlamydia trachomatis ;* ou
le cancer est situé dans le vagin et la ou les plusieurs espèces bactériennes pathogènes sont choisies parmi *Bacteroides fragilis* et *Escherichia coli* ; ou
le cancer est situé dans le sein et l'espèce bactérienne pathogène est *Staphylococcus aureus ;* ou
le cancer est situé dans la vésicule biliaire et la ou les plusieurs espèces bactériennes pathogènes sont choisies parmi *Bacteroides fragilis, Bacteroides vulgatus, Bacteroides thetaiotaomicron, Clostridium perfringens, Clostridium difficile, Escherichia coli, Salmonella enteritidis, Yersinia enterocolitica* et *Shigella flexneri ;* ou
le cancer est situé dans la vessie et l'espèce bactérienne pathogène est *Escherichia coli ;* ou
le cancer est un lymphome associé à la tête ou au cou et la ou les plusieurs espèces bactériennes pathogènes sont choisies parmi *Corynebacterium diphtheriae, Corynebacterium ulcerans, Arcanobacterium haemolyticum, Staphylococcus aureus, Pseudomonas aeruginosa, Prevotella melaninogenicus*, les streptocoques anaérobies, les streptocoques viridans et l'espèce *Actinomyces ;* ou
le cancer est un lymphome associé au thorax et la ou les plusieurs espèces bactériennes pathogènes sont choisies parmi *Streptococcus pneumoniae, Moraxella catarrhalis, Mycoplasma pneumoniae, Klebsiella pneumoniae, Haemophilus influenzae, Staphylococcus aureus, Chlamydia pneumoniae* et *Legionella pneumophila ;* ou
le cancer est un lymphome associé à la cavité abdominale et la ou les plusieurs espèces bactériennes pathogènes sont choisies parmi *Bacteroides fragilis, Bacteroides vulgatus, Bacteroides thetaiotaomicron, Clostridium perfringens, Clostridium difficile, Escherichia* coli, *Salmonella enteritidis, Yersinia enterocolitica, Shigella flexneri, Proteus mirabilis, Proteus vulgatus,* l'espèce *Providentia*, l'espèce *Morganella* et *Enterococcus faecalis ;* ou
le cancer est un lymphome associé aux zones axillaires ou inguinales et la ou les plusieurs espèces bactériennes pathogènes sont choisies parmi *Staphylococcus aureus, Corynebacterium diphtheriae, Corynebacterium ulcerans* et *Pseudomonas aeruginosa.*

3. Préparation pour une utilisation selon la revendication 1 ou utilisation selon la revendication 2, où ledit cancer est un cancer du poumon et les espèces bactériennes pathogènes sont *Streptococcus pneumoniae, Moraxella catarrhalis* ou *Mycoplasma pneumoniae.*

4. Préparation pour une utilisation selon la revendication 1 ou utilisation selon la revendication 2, où ledit cancer est un cancer du poumon, et lesdites espèces bactériennes pathogènes sont *Streptococcus pneumoniae, Moraxella catarrhalis, Klebsiella pneumoniae, Haemophilus influenzae,* ou *Staphylococcus aureus.*

5. Préparation pour une utilisation selon la revendication 1 ou utilisation selon la revendication 2, où ledit cancer est un cancer de la bouche et les espèces bactériennes pathogènes sont des streptocoques viridans choisis parmi *Streptococcus salivarius, Streptococcus sanguis,* et *Streptococcus mutans.*

6. Préparation pour une utilisation selon la revendication 1 ou utilisation selon la revendication 2, où ledit cancer est un cancer de la vésicule biliaire et l'espèce bactérienne pathogène est *Escherichia coli.*

7. Préparation pour une utilisation selon l'une quelconque des revendications précédentes ou utilisation selon l'une quelconque des revendications précédentes, où ladite espèce bactérienne pathogène est capable de provoquer une infection lorsqu'elle est présente dans ledit organe, ledit tissu ou ladite cellule spécifique chez un sujet en bonne santé.

8. Préparation pour une utilisation selon l'une quelconque des revendications précédentes ou utilisation selon l'une quelconque des revendications précédentes, où ladite espèce bactérienne pathogène a provoqué une infection dans ledit organe, ledit tissu ou ladite cellule chez un sujet en bonne santé.

9. Préparation pour une utilisation selon l'une quelconque des revendications précédentes ou utilisation selon l'une quelconque des revendications précédentes, où ledit antigène est destiné à une administration en administrant une espèce bactérienne entière.

10. Préparation pour une utilisation selon l'une quelconque des revendications précédentes ou utilisation selon l'une quelconque des revendications précédentes, où le sujet doit recevoir l'administration d'au moins deux espèces bactériennes ou plus.

11. Préparation pour une utilisation selon la revendication 10 ou utilisation selon la revendication 10, où le sujet doit recevoir l'administration d'au moins trois espèces bactériennes ou plus.

12. Préparation pour une utilisation selon l'une quelconque des revendications précédentes ou utilisation selon l'une quelconque des revendications précédentes, où le sujet doit recevoir l'administration d'un complément.

13. Préparation pour une utilisation selon l'une quelconque des revendications précédentes ou utilisation selon l'une quelconque des revendications précédentes, où le sujet doit recevoir l'administration d'un adjuvant.

14. Préparation pour une utilisation selon la revendication 1 ou utilisation selon la revendication 2, où la préparation est destinée à une administration sous-cutanée.

15. Préparation pour une utilisation selon la revendication 14, ou utilisation selon la revendication 14, où la préparation est destinée à une administration à une dose suffisante pour provoquer une réaction cutanée locale, comme une tache rose ou rouge de forme arrondie d'un diamètre d'un à trois pouces (2,54 cm à 7,62 cm) au niveau du site d'injection.

16. Préparation pour une utilisation selon la revendication 1 ou utilisation selon la revendication 2, où la préparation est destinée à une administration en plusieurs doses divisées dans le temps, comme une administration tous les deux jours ou trois fois par semaine.

17. Préparation pour une utilisation selon la revendication 16, ou utilisation selon la revendication 16, où la préparation est destinée à une administration trois fois par semaine et les doses sont administrées pendant une période d'au moins six mois.

18. Préparation pour une utilisation selon la revendication 17, ou utilisation selon la revendication 17, où la préparation est destinée à une administration sous-cutanée à une dose suffisante pour provoquer une réaction cutanée locale, comme une tache rose ou rouge de forme arrondie d'un diamètre d'un à trois pouces (2,54 cm à 7,62 cm) au niveau du site d'injection, de préférence une tache rose ou rouge de forme arrondie d'un diamètre d'un à deux pouces (2,54 cm à 5,08 cm) au niveau du site d'injection.
